(19) Europäisches Patentamt / European Patent Office / Office européen des brevets

(11) **EP 2 882 767 B1**

(12) **EUROPEAN PATENT SPECIFICATION**

(45) Date of publication and mention
of the grant of the patent:
**12.07.2023 Bulletin 2023/28**

(21) Application number: **13829632.2**

(22) Date of filing: **09.08.2013**

(51) International Patent Classification (IPC):
**C07H 5/06** (2006.01)   **A61K 31/728** (2006.01)
**C08B 37/08** (2006.01)   **A61P 13/00** (2006.01)
**A61K 49/00** (2006.01)

(52) Cooperative Patent Classification (CPC):
**A61K 31/728; A61P 13/00**

(86) International application number:
**PCT/US2013/054397**

(87) International publication number:
**WO 2014/028339 (20.02.2014 Gazette 2014/08)**

(54) **EVALUATING RENAL INJURY USING HYALURONIC ACID**

**BEWERTUNG VON NIERENLÄSIONEN MITHILFE VON HYALURONSÄURE**

**ÉVALUATION D'UNE LÉSION RÉNALE À L'AIDE DE L'ACIDE HYALURONIQUE**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.08.2012 US 201261682213 P**

(43) Date of publication of application:
**17.06.2015 Bulletin 2015/25**

(73) Proprietors:
• **Astute Medical, Inc.
Salt Lake City, UT 84108 (US)**
• **University of Pittsburgh - Of the Commonwealth
System of Higher Education
Pittsburgh, PA 15260 (US)**

(72) Inventors:
• **ANDERGERG, Joseph
Encinitas, CA 92024 (US)**
• **GRAY, Jeff
deceased (US)**
• **MCPHERSON, Paul
Encinitas, CA 92024 (US)**
• **NAKAMURA, Kevin
Encinitas
CA 92024 (US)**
• **KAMPF, James, Patrick
San Diego, CA 92130 (US)**

• **SINGBARTL, Kai
Hummelstown, PA 17036 (US)**
• **KELLUM, John, A., Jr.
Pittsburgh, PA 15206 (US)**

(74) Representative: **Wilding, James Roger et al
Mathys & Squire
The Shard
32 London Bridge Street
London SE1 9SG (GB)**

(56) References cited:
**WO-A1-2006/065604    WO-A1-2009/006244
WO-A1-2011/075744    WO-A1-2012/040592
WO-A2-2010/036342**

• **ERAY YAGMUR ET AL: "Hyaluronan serum
concentrations are elevated in critically ill
patients and associated with disease severity",
CLINICAL BIOCHEMISTRY, ELSEVIER INC, US,
CA, vol. 45, no. 1, 25 October 2011 (2011-10-25),
pages 82-87, XP028121825, ISSN: 0009-9120,
DOI: 10.1016/J.CLINBIOCHEM.2011.10.016
[retrieved on 2011-11-04]**
• **Christian Thomas ET AL: "Renal Failure",
Deutsches Aerzteblatt Online, 21 December 2009
(2009-12-21), XP055567785, DOI:
10.3238/arztebl.2009.0849**

**Description**

The present application claims priority to United States Provisional Application No. 61/682,213 filed August 11, 2012 .

BACKGROUND OF THE INVENTION

WO 2010/036342 relates to urinary biomarkers for predicting long-term dialysis. Yagmur et al., Clin. Biochem. 45(1-2): 82-87, 2012 reports that hyaluronan serum concentrations are elevated in critically ill patients and are associated with disease severity.

The term "sepsis" has been used to describe a variety of clinical conditions related to systemic manifestations of inflammation accompanied by an infection. Because of clinical similarities to inflammatory responses secondary to non-infectious etiologies, identifying sepsis has been a particularly challenging diagnostic problem. Recently, the American College of Chest Physicians and the American Society of Critical Care Medicine (Bone et al., Chest 101: 1644-53, 1992) published definitions for "Systemic Inflammatory Response Syndrome" (or "SIRS"), which refers generally to a severe systemic response to an infectious or non-infectious insult, and for the related syndromes "sepsis," "severe sepsis," and "septic shock," and extending to multiple organ dysfunction syndrome ("MODS"). These definitions, described below, are intended for each of these phrases for the purposes of the present application.

"SIRS" refers to a condition that exhibits two or more of the following:

a temperature > 38°C or < 36°C;
a heart rate of > 90 beats per minute (tachycardia);
a respiratory rate of > 20 breaths per minute (tachypnea) or a $P_aCO_2$ < 4.3 kPa; and
a white blood cell count > 12,000 per $mm^3$, < 4,000 per $mm^3$, or > 10% immature (band) forms.

"Sepsis" refers to SIRS, further accompanied by a clinically evident or microbiologically confirmed infection. This infection may be bacterial, fungal, parasitic, or viral.

"Severe sepsis" refers to a subset of sepsis patients, in which sepsis is further accompanied by organ hypoperfusion made evident by at least one sign of organ dysfunction such as hypoxemia, oliguria, metabolic acidosis, or altered cerebral function.

"Septic shock" refers to a subset of severe sepsis patients, in which severe sepsis is further accompanied by hypotension, made evident by a systolic blood pressure < 90 mm Hg, or the requirement for pharmaceutical intervention to maintain blood pressure.

MODS (multiple organ dysfunction syndrome) is the presence of altered organ function in a patient who is acutely ill such that homeostasis cannot be maintained without intervention. Primary MODS is the direct result of a well-defined insult in which organ dysfunction occurs early and can be directly attributable to the insult itself. Secondary MODS develops as a consequence of a host response and is identified within the context of SIRS.

A systemic inflammatory response leading to a diagnosis of SIRS may be related to both infection and to numerous non-infective etiologies, including burns, pancreatitis, trauma, heat stroke, and neoplasia. While conceptually it may be relatively simple to distinguish between sepsis and non-septic SIRS, no diagnostic tools have been described to unambiguously distinguish these related conditions. *See, e.g.,* Llewelyn and Cohen, Int. Care Med. 27: S10-S32, 2001. For example, because more than 90% of sepsis cases involve bacterial infection, the "gold standard" for confirming infection has been microbial growth from blood, urine, pleural fluid, cerebrospinal fluid, peritoneal fluid, synnovial fluid, sputum, or other tissue specimens. Such culture has been reported, however, to fail to confirm 50% or more of patients exhibiting strong clinical evidence of sepsis. *See, e.g.,* Jaimes et al., Int. Care Med 29: 1368-71, published electronically June 26, 2003.

Development of acute kidney injury (AKI) during sepsis increases patient morbidity, predicts higher mortality, has a significant effect on multiple organ functions, is associated with an increased length of stay in the intensive care unit, and hence consumes considerable healthcare resources. Several authors have noted that, when compared with AKI of nonseptic origin, septic AKI is characterized by a distinct pathophysiology and therefore requires a different approach. Sepsis-related AKI has been described in terms of elevated and imbalanced pro- and anti-inflammatory mediators (the so-called "peak concentration hypothesis"), coupled with severe endothelial dysfunction and a perturbed coagulation cascade operate synergistically to induce chemically and biologically mediated kidney injury. Major impediments to progress in understanding, early diagnosis, and application of appropriate therapeutic modalities in sepsis-induced AKI include limited histopathologic information, few animal models that closely mimic human sepsis, and a relative shortage of specific diagnostic tools. *See, e.g.,* Zarjou and Agarwal, J. Am. Soc. Nephrol. 22: 999-1006, 2011; Ronco et al., Clin. J. Am. Soc. Nephrol. 3: 531-44, 2008.

These limitations underscore the need for better methods to evaluate sepsis patients in order to identify those most at risk for AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

The present invention provides a method for evaluating renal status in a sepsis patient, comprising:

performing one or more assays configured to detect hyaluronic acid in a urine sample obtained from the sepsis patient to provide an assay result; and
correlating the assay result to the renal status of the sepsis patient, wherein either:

(i) the sepsis patient is in RIFLE stage 0 or R, and said correlating step comprises assigning a likelihood that the patient will develop a RIFLE I or RIFLE F acute kidney injury within 72 hours of the time the sample is obtained from the patient; or
(ii) the sepsis patient is in RIFLE stage I, and said correlating step comprises assigning a likelihood that the patient will develop a RIFLE F acute kidney injury within 72 hours of the time the sample is obtained from the patient.

Further embodiments of the invention are defined in the attached claims.

DETAILED DESCRIPTION

The technical information set out below may in some respects go beyond the scope of the invention, which is defined exclusively by the appended claims. The additional technical information is provided to place the actual invention in a broader technical context and to illustrate possible related technical developments.
It is an object of the invention to provide methods for evaluating renal status in a sepsis patient diagnosed with sepsis. As described herein, measurement of hyaluronic acid (referred to herein as a "kidney injury marker") can be used for diagnosis, prognosis, risk stratification, staging, monitoring, categorizing and determination of further diagnosis and treatment regimens in sepsis patients. In the invention, a method is performed in accordance with the attached set of claims.
The kidney injury marker used in the present invention may be used individually or in panels comprising a plurality of kidney injury markers, for risk stratification (that is, to identify sepsis patients at risk for a future injury to renal function, for future progression to reduced renal function, for future progression to ARF, for future improvement in renal function, *etc.*); for diagnosis of existing disease (that is, to identify sepsis patients who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a sepsis patient will require renal replacement therapy (i.e., hemodialysis, peritoneal dialysis, hemofiltration, and/or renal transplantation, a decreased or increased risk that a sepsis patient will recover from an injury to renal function, a decreased or increased risk that a sepsis patient will recover from ARF, a decreased or increased risk that a sepsis patient will progress to end stage renal disease, a decreased or increased risk that a sepsis patient will progress to chronic renal failure, a decreased or increased risk that a sepsis patient will suffer rejection of a transplanted kidney, *etc.*
The present invention relates to methods for evaluating renal status in a sepsis patient. These methods comprise performing an assay method that is configured to detect hyaluronic acid in a urine sample obtained from the sepsis patient. The assay result, for example a measured concentration of hyaluronic acid, is then correlated to the renal status of the sepsis patient. This correlation to renal status may include correlating the assay result(s) to one or more of risk stratification, diagnosis, prognosis, staging, classifying and monitoring of the sepsis patient as described herein. In the invention, either: (i) the sepsis patient is in RIFLE stage 0 or R, and said correlating step comprises assigning a likelihood that the patient will develop a RIFLE I or RIFLE F acute kidney injury within 72 hours of the time the sample is obtained from the patient; or (ii) the sepsis patient is in RIFLE stage I, and said correlating step comprises assigning a likelihood that the patient will develop a RIFLE F acute kidney injury within 72 hours of the time the sample is obtained from the patient.
The methods for evaluating renal status are methods for risk stratification of the sepsis patient; that is, assigning a likelihood of one or more future changes in renal status to the sepsis patient. In these embodiments, the hyaluronic acid assay result is correlated to one or more such future changes. The following are preferred risk stratification embodiments.
In preferred risk stratification embodiments, these methods comprise determining a sepsis patient's risk for a future injury to renal function, and the hyaluronic acid assay result is correlated to a likelihood of such a future injury to renal function. For example, the measured concentration may be compared to a threshold value. For a "positive going" kidney injury marker, an increased likelihood of suffering a future injury to renal function is assigned to the sepsis patient when the measured concentration is above the threshold, relative to a likelihood assigned when the measured concentration is below the threshold. For a "negative going" kidney injury marker, an increased likelihood of suffering a future injury to renal function is assigned to the sepsis patient when the measured concentration is below the threshold, relative to a likelihood assigned when the measured concentration is above the threshold.

In other preferred risk stratification embodiments, these methods comprise determining a sepsis patient's risk for future reduced renal function, and the hyaluronic acid assay result is correlated to a likelihood of such reduced renal function. For example, the measured concentration may be compared to a threshold value. For a "positive going" kidney injury marker, an increased likelihood of suffering a future reduced renal function is assigned to the sepsis patient when the measured concentration is above the threshold, relative to a likelihood assigned when the measured concentration is below the threshold. For a "negative going" kidney injury marker, an increased likelihood of future reduced renal function is assigned to the sepsis patient when the measured concentration is below the threshold, relative to a likelihood assigned when the measured concentration is above the threshold.

In still other preferred risk stratification embodiments, these methods comprise determining a sepsis patient's likelihood for a future improvement in renal function, and the hyaluronic acid assay result is correlated to a likelihood of such a future improvement in renal function. For example, the measured concentration may be compared to a threshold value. For a "positive going" kidney injury marker, an increased likelihood of a future improvement in renal function is assigned to the sepsis patient when the measured concentration is below the threshold, relative to a likelihood assigned when the measured concentration is above the threshold. For a "negative going" kidney injury marker, an increased likelihood of a future improvement in renal function is assigned to the sepsis patient when the measured concentration is above the threshold, relative to a likelihood assigned when the measured concentration is below the threshold.

In yet other preferred risk stratification embodiments, these methods comprise determining a sepsis patient's risk for progression to ARF, and the hyaluronic acid assay result is correlated to a likelihood of such progression to ARF. For example, the measured concentration may each be compared to a threshold value. For a "positive going" kidney injury marker, an increased likelihood of progression to ARF is assigned to the sepsis patient when the measured concentration is above the threshold, relative to a likelihood assigned when the measured concentration is below the threshold. For a "negative going" kidney injury marker, an increased likelihood of progression to ARF is assigned to the sepsis patient when the measured concentration is below the threshold, relative to a likelihood assigned when the measured concentration is above the threshold.

And in other preferred risk stratification embodiments, these methods comprise determining a sepsis patient's outcome risk, and the hyaluronic acid assay result is correlated to a likelihood of the occurrence of a clinical outcome related to a renal injury suffered by the sepsis patient. For example, the measured concentration may be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the sepsis patient when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the sepsis patient when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the sepsis patient. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the sepsis patient is equivalent to diagnosis of a current condition. In the invention, a likelihood is assigned that a sepsis patient in RIFLE stage 0 or R will develop a RIFLE I or RIFLE F acute kidney injury, or a likelihood that a sepsis patient in RIFLE stage I will develop a RIFLE F acute kidney injury, within 72 hours of obtaining a urine sample.

In other embodiments, the methods for evaluating renal status include diagnosing a renal injury in a sepsis patient; that is, assessing whether or not a sepsis patient has suffered from an injury to renal function, reduced renal function, or ARF. In these embodiments, the assay result, for example a measured concentration of hyaluronic acid assay, is correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the hyaluronic acid assay result is correlated to the occurrence or nonoccurrence of such an injury. For example, the measured hyaluronic acid concentration may be compared to a threshold value. For a positive going marker, an increased likelihood of the occurrence of an injury to renal function is assigned to the sepsis patient when the measured concentration is above the threshold (relative to the likelihood assigned when the measured concentration is below the threshold); alternatively, when the measured concentration is below the threshold, an increased likelihood of the nonoccurrence of an injury to renal function may be assigned to the sepsis patient (relative to the likelihood assigned when the measured concentration is above the threshold). For a negative going marker, an increased likelihood of the occurrence of an injury to renal function is assigned to the sepsis patient when the measured concentration is below the threshold (relative to the likelihood assigned when the measured concentration is above the threshold); alternatively, when the measured concentration is above the threshold, an increased likelihood of the nonoccurrence of an injury to renal function may be assigned to the sepsis patient (relative to the likelihood assigned when the measured concentration is below the threshold).

In other preferred diagnostic embodiments, these methods comprise diagnosing the occurrence or nonoccurrence of reduced renal function, and the hyaluronic acid assay result is correlated to the occurrence or nonoccurrence of an injury causing reduced renal function. For example, the measured hyaluronic acid concentration may be compared to a threshold value. For a positive going marker, an increased likelihood of the occurrence of an injury causing reduced renal function is assigned to the sepsis patient when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the sepsis patient (relative to the likelihood assigned when the measured concentration is above the threshold). For a negative going marker, an increased likelihood of the occurrence of an injury causing reduced renal function is assigned to the sepsis patient when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the sepsis patient (relative to the likelihood assigned when the measured concentration is below the threshold).

In yet other preferred diagnostic embodiments, these methods comprise diagnosing the occurrence or nonoccurrence of ARF, and the hyaluronic acid assay result is correlated to the occurrence or nonoccurrence of an injury causing ARF. For example, the measured hyaluronic acid concentration may be compared to a threshold value. For a positive going marker, an increased likelihood of the occurrence of ARF is assigned to the sepsis patient when the measured concentration is above the threshold (relative to the likelihood assigned when the measured concentration is below the threshold); alternatively, when the measured concentration is below the threshold, an increased likelihood of the nonoccurrence of ARF may be assigned to the sepsis patient (relative to the likelihood assigned when the measured concentration is above the threshold). For a negative going marker, an increased likelihood of the occurrence of ARF is assigned to the sepsis patient when the measured concentration is below the threshold (relative to the likelihood assigned when the measured concentration is above the threshold); alternatively, when the measured concentration is above the threshold, an increased likelihood of the nonoccurrence of ARF may be assigned to the sepsis patient (relative to the likelihood assigned when the measured concentration is below the threshold).

In still other preferred diagnostic embodiments, these methods comprise diagnosing a sepsis patient as being in need of renal replacement therapy, and the hyaluronic acid assay result is correlated to a need for renal replacement therapy. For example, the measured hyaluronic acid concentration may be compared to a threshold value. For a positive going marker, an increased likelihood of the occurrence of an injury creating a need for renal replacement therapy is assigned to the sepsis patient when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the sepsis patient (relative to the likelihood assigned when the measured concentration is above the threshold). For a negative going marker, an increased likelihood of the occurrence of an injury creating a need for renal replacement therapy is assigned to the sepsis patient when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the sepsis patient (relative to the likelihood assigned when the measured concentration is below the threshold).

In still other preferred diagnostic embodiments, these methods comprise diagnosing a sepsis patient as being in need of renal transplantation, and the assay result is correlated to a need for renal transplantation. For example, the measured hyaluronic acid concentration may be compared to a threshold value. For a positive going marker, an increased likelihood of the occurrence of an injury creating a need for renal transplantation is assigned to the sepsis patient when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the sepsis patient (relative to the likelihood assigned when the measured concentration is above the threshold). For a negative going marker, an increased likelihood of the occurrence of an injury creating a need for renal transplantation is assigned to the sepsis patient when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the sepsis patient (relative to the likelihood assigned when the measured concentration is below the threshold).

In still other embodiments, the methods for evaluating renal status include monitoring a renal injury in a sepsis patient; that is, assessing whether or not renal function is improving or worsening in a sepsis patient who has suffered from an injury to renal function, reduced renal function, or ARF. In these embodiments, the assay result, for example the measured concentration of hyaluronic acid, is correlated to the occurrence or nonoccurrence of a change in renal status. The following are preferred monitoring embodiments.

In preferred monitoring embodiments, these methods comprise monitoring renal status in a sepsis patient suffering from an injury to renal function, and the hyaluronic acid assay result is correlated to the occurrence or nonoccurrence of a change in renal status in the sepsis patient. For example, the measured hyaluronic acid concentration may be compared to a threshold value. For a positive going marker, when the measured concentration is above the threshold, a worsening of renal function may be assigned to the sepsis patient; alternatively, when the measured concentration is below the threshold, an improvement of renal function may be assigned to the sepsis patient. For a negative going marker, when the measured concentration is below the threshold, a worsening of renal function may be assigned to the sepsis patient; alternatively, when the measured concentration is above the threshold, an improvement of renal function may be assigned to the sepsis patient.

In other preferred monitoring embodiments, these methods comprise monitoring renal status in a sepsis patient suffering from reduced renal function, and the hyaluronic acid assay result is correlated to the occurrence or nonoccurrence of a change in renal status in the sepsis patient. For example, the measured hyaluronic acid concentration may be compared to a threshold value. For a positive going marker, when the measured concentration is above the threshold, a worsening of renal function may be assigned to the sepsis patient; alternatively, when the measured concentration is below the threshold, an improvement of renal function may be assigned to the sepsis patient. For a negative going marker, when the measured concentration is below the threshold, a worsening of renal function may be assigned to the sepsis patient; alternatively, when the measured concentration is above the threshold, an improvement of renal function may be assigned to the sepsis patient.

In yet other preferred monitoring embodiments, these methods comprise monitoring renal status in a sepsis patient suffering from acute renal failure, and the hyaluronic acid assay result is correlated to the occurrence or nonoccurrence of a change in renal status in the sepsis patient. For example, the measured hyaluronic acid concentration may be compared to a threshold value. For a positive going marker, when the measured concentration is above the threshold, a worsening of renal function may be assigned to the sepsis patient; alternatively, when the measured concentration is below the threshold, an improvement of renal function may be assigned to the sepsis patient. For a negative going marker, when the measured concentration is below the threshold, a worsening of renal function may be assigned to the sepsis patient; alternatively, when the measured concentration is above the threshold, an improvement of renal function may be assigned to the sepsis patient.

In still other embodiments, the methods for evaluating renal status include classifying a renal injury in a sepsis patient; that is, determining whether a renal injury in a sepsis patient is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a sepsis patient will progress to a particular RIFLE stage. In these embodiments, the assay result, for example the measured concentration of hyaluronic acid, is correlated to a particular class and/or subclass. The following are preferred classification embodiments.

In preferred classification embodiments, these methods comprise determining whether a renal injury in a sepsis patient is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a sepsis patient will progress to a particular RIFLE stage, and the hyaluronic acid assay result is correlated to the injury classification for the sepsis patient. For example, the measured concentration may be compared to a threshold value, and when the measured concentration is above the threshold, a particular classification is assigned; alternatively, when the measured concentration is below the threshold, a different classification may be assigned to the sepsis patient.

A variety of methods may be used by the skilled artisan to arrive at a desired threshold value for use in these methods. For example, the threshold value may be determined from a population of normal sepsis patients by selecting a concentration representing the 75th, 85th, 90th, 95th, or 99th percentile of a kidney injury marker measured in such normal sepsis patients. Alternatively, the threshold value may be determined from a "diseased" population of sepsis patients, e.g., those suffering from an injury or having a predisposition for an injury (e.g., progression to ARF or some other clinical outcome such as death, dialysis, renal transplantation, *etc.),* by selecting a concentration representing the 75th, 85th, 90th, 95th, or 99th percentile of a kidney injury marker measured in such sepsis patients. In another alternative, the threshold value may be determined from a prior measurement of a kidney injury marker in the same sepsis patient; that is, a temporal change in the level of a kidney injury marker in the sepsis patient may be used to assign risk to the sepsis patient.

The foregoing discussion is not meant to imply, however, that the kidney injury markers must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the sepsis patient, the occurrence of an injury, a classification, etc. In yet another alternative, a threshold that can provide an acceptable level of specificity and sensitivity in separating a population of sepsis patients into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/-5% of a given measurement.
Multiple thresholds may also be used to assess renal status in a sepsis patient. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to sepsis patients based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the methods of the invention, a urine sample is used.

The foregoing method steps should not be interpreted to mean that the hyaluronic acid assay result is used in isolation in the methods described herein. Rather, additional variables or other clinical indicia may be included in the methods described herein. For example, a risk stratification, diagnostic, classification, monitoring, etc. method may combine the assay result(s) with one or more variables measured for the sepsis patient selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815 .

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

Devices and kits may be used for performing the methods described herein. Suitable kits comprise reagents sufficient for performing an assay for at least one of the described kidney injury markers, together with instructions for performing the described threshold comparisons. Devices and kits are not claimed as such.

Reagents for performing such assays may be provided in an assay device, and such assay devices may be included in such a kit. Preferred reagents can comprise one or more solid phase antibodies, the solid phase antibody comprising antibody that detects the intended biomarker target bound to a solid support. In the case of sandwich immunoassays, such reagents can also include one or more detectably labeled antibodies, the detectably labeled antibody comprising antibody that detects the intended biomarker target bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.)* as well as molecules that may be indirectly detected by production of a detectable reaction product (*e.g.,* enzymes such as horseradish peroxidase, alkaline phosphatase, *etc.)* or through the use of a specific binding molecule which itself may be detectable (*e.g.,* a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.,* a diffraction grating, electrochemical sensor, etc) for generation of a signal, while in others, a signal is generated by a transducer that is spatially separate from the solid phase antibody (*e.g.,* a fluorometer that employs an excitation light source and an optical detector). This list is not meant to be limiting. Antibody-based biosensors may also be employed to determine the presence or amount of analytes that optionally eliminate the need for a labeled molecule.

FURTHER DETAILED DESCRIPTION

The present disclosure relates to methods and compositions for diagnosis, differential diagnosis, risk stratification, monitoring, classifying and determination of treatment regimens in sepsis patients diagnosed with sepsis. In various instances, a measured concentration of hyaluronic acis is correlated to the renal status of the sepsis patient. The present invention relates to methods for evaluating renal status in a sepsis patient as defined in the attached set of claims.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830 .

Renal disease and/or injury may be acute or chronic. Acute and chronic kidney disease are described as follows (from Current Medical Diagnosis & Treatment 2008, 47th Ed, McGraw Hill, New York, pages 785-815):

"Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222,:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |

(continued)

| Intrinsic Renal | |
|---|---|
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulointerstitial | Drug reaction (eg, β-lactams, NSAIDs, sulfonamides, |
| **Type** | **Risk Factors** |
| nephritis | ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of sepsis patients with ARF may be as low as about 60%.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005 .

As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004)

investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004

proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.

More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006;7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

The following is a brief description of the kidney injury marker used in the present invention.

Hyaluronic acid (HA) is a ubiquitous connective tissue glycosaminoglycan that in vivo is present as a high molecular mass component of most extracellular matrices. Although HA is not a major constituent of the normal renal corticointerstitium,3 it is expressed around renal proximal tubular epithelial cells (PTC) after both acute and chronic renal injury that is caused by numerous diseases.4, 5 Furthermore, increased deposition of interstitial HA correlates with both proteinuria and renal function in progressive renal disease.6 Binding of HA to its principle receptor, CD44, promotes inflammation through interaction between HA and CD44, expressed on inflammatory cells.7 HA/CD44 binding activates the mitogen-activated protein kinase (MAPK) pathway and enhances PTC migration, a process that is implicated in epithelial cell-fibroblast transdifferentiation and progressive renal fibrosis.8 In ischemic kidneys from diabetic subjects, the renal HA-content started to increases already after 24 hours and significantly so 1-8 weeks after ischemia/reperfusion (I/R).

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

The skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quatitation). This list is not meant to be limiting.

The term "positive going" marker as that term is used herein refer to a marker that is determined to be elevated in sepsis patients suffering from a disease or condition, relative to sepsis patients not suffering from that disease or condition. The term "negative going" marker as that term is used herein refer to a marker that is determined to be reduced in sepsis patients suffering from a disease or condition, relative to sepsis patients not suffering from that disease or condition.

The term "sepsis patient" as used herein refers to a human or non-human organism. Thus, the methods and compositions described herein are applicable to both human and veterinary disease. Further, while a sepsis patient is preferably a living organism, the disclosure described herein may be used in post-mortem analysis as well. Preferred sepsis patients are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a sepsis patient, or may be obtained from biological materials intended to be provided to the sepsis patient. For example, a sample may be obtained from a kidney being evaluated for possible transplantation into a sepsis patient, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a sepsis patient of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components. In the methods of the invention, a urine sample is used.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker , optionally together with other clinical characteristics, to arrive at a diagnosis (that is, the occurrence or nonoccurrence) of an acute renal injury or ARF for the sepsis patient from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the sepsis patient relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377 .

One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.,* fluorescent moieties, electrochemical labels, metal chelates, *etc.)* as well as molecules that may be indirectly detected by production of a detectable reaction product (*e.g.,* enzymes such as horseradish peroxidase, alkaline phosphatase, *etc.)* or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present disclosure provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits. Kits are not claimed as such.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker . The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098 .

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, *etc.,* assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a sepsis patient belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815 .

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present disclosure may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention as defined in the attached claims.

Example 1: Septic sepsis patient sample collection

The objective of this study was to collect samples from patients expected to be in the ICU for at least 48 hours were enrolled. To be enrolled in the study, each patient must meet all of the following inclusion criteria and none of the following exclusion criteria:

Inclusion Criteria: males and females 18 years of age or older and which either acquire sepsis or have sepsis on admission.

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after

contrast administration (if applicable); at 12 ($\pm$ 1), 24 ($\pm$ 2), and 48 ($\pm$ 2) hours after enrollment, and thereafter daily up to day 7 to day 14 while the sepsis patient is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 2. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Example 3. Use of Kidney Injury Markers for evaluating sepsis patients

Patients from the sepsis study (Example 1) were classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the sepsis patient is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff') concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Fig. 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

Hyaluronic acid

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1280 | 1840 | 1280 | 1890 | 1280 | 2020 |
| Average | 1710 | 2210 | 1710 | 2310 | 1710 | 2500 |
| Stdev | 1240 | 1610 | 1240 | 1630 | 1240 | 1690 |
| p(t-test) | | 0.031 | | 0.0077 | | 0.0074 |
| Min | 154 | 151 | 154 | 77.8 | 154 | 317 |
| Max | 5730 | 5710 | 5730 | 5910 | 5730 | 5450 |
| n (Samp) | 167 | 41 | 167 | 47 | 167 | 23 |
| n (Patient) | 64 | 41 | 64 | 47 | 64 | 23 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1590 | 1620 | 1590 | 1900 | 1590 | 2020 |
| Average | 2060 | 1900 | 2060 | 2170 | 2060 | 2340 |
| Stdev | 1470 | 1540 | 1470 | 1560 | 1470 | 1770 |
| p(t-test) | | 0.69 | | 0.73 | | 0.50 |
| Min | 89.4 | 151 | 89.4 | 77.8 | 89.4 | 487 |
| Max | 6350 | 6400 | 6350 | 5710 | 6350 | 5910 |
| n (Samp) | 340 | 15 | 340 | 20 | 340 | 13 |
| n (Patient) | 110 | 15 | 110 | 20 | 110 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1350 | 2280 | 1350 | 2500 | 1350 | 1500 |
| Average | 1700 | 2690 | 1700 | 2740 | 1700 | 2460 |
| Stdev | 1270 | 1780 | 1270 | 1800 | 1270 | 1810 |
| p(t-test) | | 5.4E-5 | | 1.1E-5 | | 0.014 |
| Min | 189 | 168 | 189 | 129 | 189 | 183 |
| Max | 5540 | 6400 | 5540 | 6390 | 5540 | 6190 |
| n (Samp) | 165 | 42 | 165 | 49 | 165 | 22 |
| n (Patient) | 57 | 42 | 57 | 49 | 57 | 22 |

**EP 2 882 767 B1**

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| ADC | 0.59 | 0.48 | 0.68 | 0.60 | 0.52 | 0.67 | 0.63 | 0.54 | 0.61 |
| SE | 0.051 | 0.077 | 0.049 | 0.048 | 0.067 | 0.046 | 0.066 | 0.083 | 0.067 |
| p | 0.071 | 0.79 | 3.0E-4 | 0.043 | 0.78 | 3.5E-4 | 0.056 | 0.64 | 0.11 |
| nCohort 1 | 167 | 340 | 165 | 167 | 340 | 165 | 167 | 340 | 165 |
| nCohort 2 | 41 | 15 | 42 | 47 | 20 | 49 | 23 | 13 | 22 |
| Cutoff 1 | 1130 | 1310 | 1760 | 1120 | 1250 | 1260 | 1220 | 828 | 1220 |
| Sens 1 | 71% | 73% | 71% | 70% | 70% | 71% | 74% | 77% | 73% |
| Spec 1 | 38% | 41% | 64% | 37% | 39% | 47% | 45% | 20% | 44% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 875 | 893 | 1060 | 874 | 747 | 1060 | 820 | 747 | 842 |
| Sens 2 | 80% | 80% | 81% | 81% | 80% | 82% | 83% | 85% | 82% |
| Spec 2 | 22% | 22% | 38% | 22% | 18% | 38% | 20% | 18% | 26% |
| Cutoff 3 | 346 | 168 | 462 | 394 | 458 | 437 | 705 | 658 | 705 |
| Sens 3 | 90% | 93% | 90% | 91% | 90% | 92% | 91% | 92% | 91% |
| Spec 3 | 5% | 1% | 12% | 5% | 8% | 11% | 19% | 15% | 25% |
| Cutoff 4 | 1870 | 2520 | 1940 | 1870 | 2520 | 1940 | 1870 | 2520 | 1940 |
| Sens 4 | 49% | 27% | 62% | 51% | 40% | 57% | 52% | 38% | 45% |
| Spec 4 | 70% | 70% | 70% | 70% | 70% | 70% | 70% | 70% | 70% |
| Cutoff 5 | 2410 | 3510 | 2510 | 2410 | 3510 | 2510 | 2410 | 3510 | 2510 |
| Sens 5 | 37% | 7% | 40% | 45% | 15% | 49% | 43% | 23% | 41% |
| Spec 5 | 80% | 80% | 80% | 80% | 80% | 80% | 80% | 80% | 80% |
| Cutoff 6 | 3720 | 4510 | 3720 | 3720 | 4510 | 3720 | 3720 | 4510 | 3720 |
| Sens 6 | 17% | 7% | 26% | 23% | 10% | 35% | 30% | 23% | 27% |
| Spec 6 | 90% | 90% | 90% | 90% | 90% | 90% | 90% | 90% | 90% |
| OR Quart 2 | 0.26 | 3.1 | 1.2 | 0.98 | 0.79 | 1.7 | 0.56 | 0.49 | 2.1 |
| p Value | 0.049 | 0.17 | 0.78 | 0.96 | 0.73 | 0.33 | 0.45 | 0.42 | 0.32 |
| 95% CI of | 0.066 | 0.62 | 0.34 | 0.37 | 0.21 | 0.60 | 0.13 | 0.087 | 0.49 |
| OR Quart2 | 1.00 | 16 | 4.2 | 2.6 | 3.0 | 4.7 | 2.5 | 2.7 | 8.9 |
| OR Quart 3 | 1.3 | 1.5 | 3.1 | 0.55 | 1.0 | 1.0 | 1.0 | 1.0 | 1.3 |
| p Value | 0.63 | 0.65 | 0.049 | 0.28 | 1.0 | 1.0 | 1.0 | 1.0 | 0.72 |
| 95% CI of | 0.49 | 0.25 | 1.0 | 0.18 | 0.28 | 0.32 | 0.27 | 0.24 | 0.28 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart3 | 3.2 | 9.3 | 9.4 | 1.6 | 3.6 | 3.1 | 3.7 | 4.1 | 6.3 |
| OR Quart 4 | 1.9 | 2.1 | 4.9 | 2.7 | 1.2 | 5.3 | 2.2 | 0.73 | 3.4 |
| p Value | 0.18 | 0.41 | 0.0043 | 0.025 | 0.76 | 7.0E-4 | 0.18 | 0.69 | 0.082 |
| 95% CI of | 0.75 | 0.37 | 1.6 | 1.1 | 0.36 | 2.0 | 0.69 | 0.16 | 0.86 |
| OR Quart4 | 4.6 | 12 | 14 | 6.6 | 4.1 | 14 | 7.0 | 3.4 | 13 |

Fig. 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

Hyaluronic acid

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1350 | 2480 | 1350 | 3180 | 1350 | 2210 |
| Average | 1790 | 2970 | 1790 | 3290 | 1790 | 2760 |
| Stdev | 1320 | 1920 | 1320 | 1700 | 1320 | 1890 |
| p(t-test) |  | 1.5E-4 |  | 3.8E-8 |  | 0.0054 |
| Min | 77.8 | 89.4 | 77.8 | 491 | 77.8 | 324 |
| Max | 5730 | 6400 | 5730 | 6400 | 5730 | 6190 |
| n (Samp) | 321 | 21 | 321 | 28 | 321 | 16 |
| n (Patient) | 109 | 21 | 109 | 28 | 109 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1620 | 2480 | 1620 | 2230 | 1620 | 2200 |
| Average | 2120 | 2600 | 2120 | 2960 | 2120 | 2560 |
| Stdev | 1540 | 1840 | 1540 | 1970 | 1540 | 1980 |
| p(t-test) |  | 0.41 |  | 0.11 |  | 0.35 |
| Min | 77.8 | 404 | 77.8 | 491 | 77.8 | 324 |
| Max | 6400 | 6400 | 6400 | 6400 | 6400 | 6400 |
| n (Samp) | 413 | 7 | 413 | 9 | 413 | 11 |
| n (Patient) | 133 | 7 | 133 | 9 | 133 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1390 | 3100 | 1390 | 3200 | 1390 | 3430 |
| Average | 1780 | 3340 | 1780 | 3540 | 1780 | 3250 |
| Stdev | 1350 | 1820 | 1350 | 1670 | 1350 | 1880 |
| p(t-test) | | 5.9E-7 | | 3.5E-10 | | 1.1E-4 |
| Min | 77.8 | 89.4 | 77.8 | 770 | 77.8 | 379 |
| Max | 5540 | 6400 | 5540 | 6400 | 5540 | 6190 |
| n (Samp) | 293 | 22 | 293 | 28 | 293 | 14 |
| n (Patient) | 92 | 22 | 92 | 28 | 92 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| ADC | 0.69 | 0.61 | 0.75 | 0.76 | 0.64 | 0.81 | 0.65 | 0.57 | 0.73 |
| SE | 0.066 | 0.11 | 0.061 | 0.054 | 0.10 | 0.051 | 0.076 | 0.091 | 0.078 |
| p | 0.0046 | 0.35 | 3.8E-5 | 8.7E-7 | 0.18 | 1.8E-9 | 0.043 | 0.46 | 0.0028 |
| nCohort 1 | 321 | 413 | 293 | 321 | 413 | 293 | 321 | 413 | 293 |
| nCohort 2 | 21 | 7 | 22 | 28 | 9 | 28 | 16 | 11 | 14 |
| Cutoff 1 | 1700 | 2130 | 2420 | 2170 | 1770 | 2560 | 1310 | 1310 | 1890 |
| Sens 1 | 71% | 71% | 73% | 71% | 78% | 71% | 75% | 73% | 71% |
| Spec 1 | 60% | 63% | 76% | 73% | 54% | 78% | 49% | 41% | 66% |
| Cutoff 2 | 1610 | 1610 | 1700 | 1770 | 1150 | 2120 | 1260 | 1260 | 1320 |
| Sens 2 | 81% | 86% | 82% | 82% | 89% | 82% | 81% | 82% | 86% |
| Spec 2 | 59% | 50% | 59% | 62% | 33% | 72% | 47% | 39% | 49% |
| Cutoff 3 | 394 | 394 | 874 | 781 | 477 | 1330 | 373 | 757 | 884 |
| Sens 3 | 90% | 100% | 91% | 93% | 100% | 93% | 94% | 91% | 93% |
| Spec 3 | 7% | 6% | 28% | 23% | 9% | 49% | 7% | 19% | 29% |
| Cutoff 4 | 2050 | 2650 | 2060 | 2050 | 2650 | 2060 | 2050 | 2650 | 2060 |
| Sens 4 | 67% | 14% | 77% | 71% | 44% | 82% | 50% | 27% | 57% |
| Spec 4 | 70% | 70% | 70% | 70% | 70% | 70% | 70% | 70% | 70% |
| Cutoff 5 | 2740 | 3530 | 2740 | 2740 | 3530 | 2740 | 2740 | 3530 | 2740 |
| Sens 5 | 43% | 14% | 59% | 61% | 33% | 68% | 44% | 27% | 57% |
| Spec 5 | 80% | 80% | 80% | 80% | 80% | 80% | 80% | 80% | 80% |
| Cutoff 6 | 3750 | 4590 | 3890 | 3750 | 4590 | 3890 | 3750 | 4590 | 3890 |
| Sens 6 | 38% | 14% | 45% | 39% | 22% | 36% | 31% | 18% | 43% |
| Spec 6 | 90% | 90% | 90% | 90% | 90% | 90% | 90% | 90% | 90% |
| OR Quart 2 | 0.32 | 1.0 | 0.49 | 0.33 | 0.99 | 0.49 | 2.0 | 1.5 | 2.0 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.33 | 1.0 | 0.56 | 0.34 | 0.99 | 0.57 | 0.41 | 0.65 | 0.57 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.033 | 0.062 | 0.043 | 0.033 | 0.061 | 0.044 | 0.37 | 0.25 | 0.18 |
| OR Quart2 | 3.2 | 16 | 5.5 | 3.2 | 16 | 5.6 | 12 | 9.3 | 23 |
| OR Quart 3 | 2.5 | 4.1 | 3.1 | 2.1 | 3.1 | 3.2 | 1.5 | 1.5 | 3.0 |
| p Value | 0.20 | 0.21 | 0.17 | 0.31 | 0.34 | 0.17 | 0.65 | 0.65 | 0.34 |
| 95% CI of | 0.61 | 0.45 | 0.61 | 0.50 | 0.31 | 0.62 | 0.25 | 0.25 | 0.31 |
| OR Quart3 | 9.8 | 37 | 16 | 8.6 | 30 | 16 | 9.3 | 9.3 | 30 |
| OR Quart 4 | 3.6 | 1.0 | 7.5 | 7.2 | 4.1 | 12 | 3.7 | 1.5 | 8.7 |
| p Value | 0.059 | 1.0 | 0.0097 | 0.0022 | 0.21 | 0.0011 | 0.11 | 0.65 | 0.044 |
| 95% CI of | 0.95 | 0.062 | 1.6 | 2.0 | 0.45 | 2.7 | 0.74 | 0.25 | 1.1 |
| OR Quart4 | 14 | 16 | 34 | 25 | 37 | 53 | 18 | 9.3 | 71 |

Fig. 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Hyaluronic acid**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1530 | 3070 | 1530 | 2230 | 1800 | 2890 |
| Average | 1810 | 3030 | 2240 | 2330 | 1950 | 3310 |
| Stdev | 1240 | 1850 | 1760 | 1890 | 1290 | 1800 |
| p(t-test) | | 0.0044 | | 0.90 | | 0.013 |
| Min | 151 | 183 | 151 | 183 | 168 | 1240 |
| Max | 5180 | 6190 | 6400 | 5250 | 5180 | 6400 |
| n (Samp) | 41 | 18 | 19 | 9 | 31 | 10 |
| n (Patient) | 41 | 18 | 19 | 9 | 31 | 10 |

|  | At Enrollment | | |
|---|---|---|---|
|  | sCr or UO | sCr only | UO only |
| ADC | 0.71 | 0.49 | 0.74 |
| SE | 0.078 | 0.12 | 0.099 |
| p | 0.0080 | 0.90 | 0.015 |
| nCohort 1 | 41 | 19 | 31 |
| nCohort 2 | 18 | 9 | 10 |
| Cutoff 1 | 1800 | 842 | 2120 |
| Sens 1 | 72% | 78% | 70% |
| Spec 1 | 61% | 21% | 65% |
| Cutoff 2 | 1140 | 183 | 1800 |
| Sens 2 | 83% | 89% | 80% |
| Spec 2 | 34% | 5% | 52% |
| Cutoff 3 | 272 | 151 | 1530 |
| Sens 3 | 94% | 100% | 90% |
| Spec 3 | 7% | 5% | 39% |
| Cutoff 4 | 2120 | 2780 | 2320 |
| Sens 4 | 67% | 33% | 60% |
| Spec 4 | 71% | 74% | 71% |

|  | At Enrollment | | |
|---|---|---|---|
|  | sCr or UO | sCr only | UO only |
| Cutoff 5 | 2700 | 3330 | 2560 |
| Sens 5 | 56% | 22% | 60% |
| Spec 5 | 80% | 84% | 81% |
| Cutoff 6 | 3330 | 6190 | 3530 |
| Sens 6 | 33% | 0% | 30% |
| Spec 6 | 90% | 95% | 90% |
| OR Quart 2 | 0.56 | 0.53 | >4.3 |
| p Value | 0.57 | 0.58 | <0.25 |
| 95% CI of | 0.079 | 0.058 | >0.37 |
| OR Quart2 | 4.0 | 4.9 | na |
| OR Quart 3 | 0.92 | 0.22 | >2.5 |
| p Value | 0.92 | 0.26 | <0.49 |
| 95% CI of | 0.15 | 0.017 | >0.19 |
| OR Quart3 | 5.5 | 3.0 | na |
| OR Quart 4 | 7.3 | 1.0 | >8.3 |
| p Value | 0.019 | 1.0 | <0.080 |
| 95% CI of | 1.4 | 0.12 | >0.78 |

(continued)

|  | At Enrollment | | |
|---|---|---|---|
|  | sCr or UO | sCr only | UO only |
| OR Quart4 | 39 | 8.3 | na |

Fig. 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

Hyaluronic acid

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1860 | 3670 | 1860 | 3670 | 1860 | 3370 |
| Average | 2170 | 3890 | 2170 | 3850 | 2170 | 3550 |
| Stdev | 1380 | 1500 | 1380 | 1510 | 1380 | 1310 |
| p(t-test) | 4.3E-6 | 4.3E-6 |  | 6.6E-6 |  | 0.0019 |
| Min | 324 | 823 | 324 | 580 | 324 | 1330 |
| Max | 5730 | 6400 | 5730 | 6400 | 5730 | 6190 |
| n (Samp) | 64 | 22 | 64 | 22 | 64 | 12 |
| n (Patient) | 64 | 22 | 64 | 22 | 64 | 12 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2110 | 3740 | 2110 | 3170 | 2110 | 3170 |
| Average | 2610 | 3550 | 2610 | 3220 | 2610 | 3130 |
| Stdev | 1560 | 1770 | 1560 | 1520 | 1560 | 1130 |
| p(t-test) |  | 0.090 |  | 0.27 |  | 0.47 |
| Min | 324 | 823 | 324 | 580 | 324 | 1330 |
| Max | 6350 | 6400 | 6350 | 5000 | 6350 | 4360 |
| n (Samp) | 110 | 9 | 110 | 9 | 110 | 5 |
| n (Patient) | 110 | 9 | 110 | 9 | 110 | 5 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1860 | 3600 | 1860 | 3600 | 1860 | 3210 |
| Average | 2150 | 4090 | 2150 | 4060 | 2150 | 3470 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 1390 | 1520 | 1390 | 1510 | 1390 | 1330 |
| p(t-test) | | 2.2E-6 | | 2.8E-6 | | 0.0038 |
| Min | 324 | 1330 | 324 | 1330 | 324 | 1330 |
| Max | 5540 | 6400 | 5540 | 6400 | 5540 | 6190 |
| n (Samp) | 57 | 19 | 57 | 19 | 57 | 12 |
| n (Patient) | 57 | 19 | 57 | 19 | 57 | 12 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| ADC | 0.80 | 0.65 | 0.83 | 0.80 | 0.62 | 0.83 | 0.78 | 0.63 | 0.77 |
| SE | 0.061 | 0.10 | 0.061 | 0.061 | 0.10 | 0.061 | 0.083 | 0.14 | 0.084 |
| p | 7.2E-7 | 0.15 | 4.3E-8 | 1.1E-6 | 0.25 | 6.8E-8 | 6.8E-4 | 0.36 | 0.0012 |
| nCohort 1 | 64 | 110 | 57 | 64 | 110 | 57 | 64 | 110 | 57 |
| nCohort 2 | 22 | 9 | 19 | 22 | 9 | 19 | 12 | 5 | 12 |
| Cutoff 1 | 3050 | 2690 | 3050 | 3050 | 2690 | 3050 | 2950 | 2950 | 2720 |
| Sens 1 | 73% | 78% | 74% | 73% | 78% | 74% | 75% | 80% | 75% |
| Spec 1 | 77% | 59% | 77% | 77% | 59% | 77% | 77% | 66% | 74% |
| Cutoff 2 | 2720 | 1280 | 2720 | 2720 | 1280 | 2720 | 2520 | 2950 | 2720 |
| Sens 2 | 82% | 89% | 84% | 82% | 89% | 84% | 83% | 80% | 83% |
| Spec 2 | 70% | 24% | 74% | 70% | 24% | 74% | 70% | 66% | 74% |
| Cutoff 3 | 2170 | 686 | 2170 | 2170 | 541 | 2170 | 2170 | 1280 | 2170 |
| Sens 3 | 91% | 100% | 95% | 91% | 100% | 95% | 92% | 100% | 92% |
| Spec 3 | 64% | 7% | 63% | 64% | 5% | 63% | 64% | 24% | 63% |
| Cutoff 4 | 2520 | 3310 | 2520 | 2520 | 3310 | 2520 | 2520 | 3310 | 2520 |
| Sens 4 | 86% | 56% | 89% | 86% | 44% | 89% | 83% | 40% | 83% |
| Spec 4 | 70% | 70% | 70% | 70% | 70% | 70% | 70% | 70% | 70% |
| Cutoff 5 | 3310 | 4160 | 3310 | 3310 | 4160 | 3310 | 3310 | 4160 | 3310 |
| Sens 5 | 59% | 44% | 58% | 59% | 33% | 58% | 50% | 20% | 42% |
| Spec 5 | 81% | 80% | 81% | 81% | 80% | 81% | 81% | 80% | 81% |
| Cutoff 6 | 4400 | 5180 | 4690 | 4400 | 5180 | 4690 | 4400 | 5180 | 4690 |
| Sens 6 | 41% | 11% | 42% | 36% | 0% | 37% | 25% | 0% | 17% |
| Spec 6 | 91% | 90% | 91% | 91% | 90% | 91% | 91% | 90% | 91% |
| OR Quart 2 | 0.95 | 0 | >2.2 | 0.95 | 0 | >2.2 | >1.1 | 0 | >1.1 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.97 | na | <0.53 | 0.97 | na | <0.53 | <0.97 | na | <0.97 |
| 95% CI of | 0.056 | na | >0.19 | 0.056 | na | >0.19 | >0.061 | na | >0.061 |
| OR Quart2 | 16 | na | na | 16 | na | na | na | na | na |
| OR Quart 3 | 15 | 1.5 | >14 | 15 | 2.1 | >14 | >6.8 | 3.1 | >9.3 |
| p Value | 0.015 | 0.67 | <0.020 | 0.015 | 0.42 | <0.020 | <0.096 | 0.34 | <0.052 |
| 95% CI of OR Quart3 | 1.7 | 0.23 | >1.5 | 1.7 | 0.35 | >1.5 | >0.71 | 0.30 | >0.98 |
| | 130 | 9.7 | na | 130 | 12 | na | na | 32 | na |
| OR Quart 4 | 20 | 2.1 | >17 | 20 | 1.5 | >17 | >8.8 | 0.96 | >6.5 |
| p Value | 0.0070 | 0.42 | <0.012 | 0.0070 | 0.67 | <0.012 | <0.057 | 0.98 | <0.10 |
| 95% CI of | 2.3 | 0.35 | >1.9 | 2.3 | 0.23 | >1.9 | >0.94 | 0.057 | >0.68 |
| OR Quart4 | 180 | 12 | na | 180 | 9.7 | na | na | 16 | na |

Fig. 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Hyaluronic acid**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 278 | 219 | 278 | 391 | 278 | 488 |
| Average | 763 | 833 | 763 | 1090 | 763 | 1140 |
| Stdev | 964 | 1150 | 964 | 1260 | 964 | 1220 |
| p(t-test) | | 0.91 | . | 0.43 | | 0.43 |
| Min | 140 | 125 | 140 | 112 | 140 | 172 |
| Max | 3200 | 2150 | 3200 | 3200 | 3200 | 3200 |
| n (Samp) | 17 | 3 | 17 | 12 | 17 | 7 |
| n (Patient) | 16 | 3 | 16 | 12 | 16 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 396 | 474 | 396 | 239 |
| Average | nd | nd | 1170 | 474 | 1170 | 239 |
| Stdev | nd | nd | 1210 | 204 | 1210 | 179 |
| p(t-test) | nd | nd | | 0.43 | | 0.29 |
| Min | nd | nd | 140 | 330 | 140 | 112 |
| Max | nd | nd | 3200 | 618 | 3200 | 365 |
| n (Samp) | nd | nd | 40 | 2 | 40 | 2 |
| n (Patient) | nd | nd | 32 | 2 | 32 | 2 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 283 | 427 | 283 | 449 | 283 | 488 |
| Average | 620 | 934 | 620 | 1050 | 620 | 1140 |
| Stdev | 761 | 1060 | 761 | 1220 | 761 | 1220 |
| p(t-test) | | 0.54 | | 0.24 | | 0.21 |
| Min | 140 | 219 | 140 | 112 | 140 | 172 |
| Max | 3200 | 2150 | 3200 | 3200 | 3200 | 3200 |
| n (Samp) | 18 | 3 | 18 | 13 | 18 | 7 |
| n (Patient) | 16 | 3 | 16 | 13 | 16 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| ADC | 0.41 | nd | 0.65 | 0.56 | 0.51 | 0.58 | 0.64 | 0.25 | 0.67 |
| SE | 0.19 | nd | 0.19 | 0.11 | 0.21 | 0.11 | 0.13 | 0.20 | 0.13 |
| p | 0.64 | nd | 0.43 | 0.58 | 0.95 | 0.43 | 0.27 | 0.22 | 0.18 |
| nCohort 1 | 17 | nd | 18 | 17 | 40 | 18 | 17 | 40 | 18 |
| nCohort 2 | 3 | nd | 3 | 12 | 2 | 13 | 7 | 2 | 7 |
| Cutoff 1 | 0 | nd | 212 | 212 | 322 | 212 | 343 | 0 | 343 |
| Sens 1 | 100% | nd | 100% | 75% | 100% | 77% | 71% | 100% | 71% |
| Spec 1 | 0% | nd | 33% | 35% | 45% | 33% | 65% | 0% | 67% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 2 | 0 | nd | 212 | 168 | 322 | 168 | 278 | 0 | 287 |
| Sens 2 | 100% | nd | 100% | 83% | 100% | 85% | 86% | 100% | 86% |
| Spec 2 | 0% | nd | 33% | 12% | 45% | 11% | 53% | 0% | 56% |
| Cutoff 3 | 0 | nd | 212 | 140 | 322 | 140 | 168 | 0 | 168 |
| Sens 3 | 100% | nd | 100% | 92% | 100% | 92% | 100% | 100% | 100% |
| Spec 3 | 0% | nd | 33% | 6% | 45% | 6% | 12% | 0% | 11% |
| Cutoff 4 | 578 | nd | 578 | 578 | 1660 | 578 | 578 | 1660 | 578 |
| Sens 4 | 33% | nd | 33% | 42% | 0% | 38% | 43% | 0% | 43% |
| Spec 4 | 71% | nd | 72% | 71% | 70% | 72% | 71% | 70% | 72% |
| Cutoff 5 | 1030 | nd | 933 | 1030 | 2900 | 933 | 1030 | 2900 | 933 |
| Sens 5 | 33% | nd | 33% | 33% | 0% | 31% | 29% | 0% | 29% |
| Spec 5 | 82% | nd | 83% | 82% | 80% | 83% | 82% | 80% | 83% |
| Cutoff 6 | 2980 | nd | 1660 | 2980 | 3200 | 1660 | 2980 | 3200 | 1660 |
| Sens 6 | 0% | nd | 33% | 17% | 0% | 23% | 14% | 0% | 29% |
| Spec 6 | 94% | nd | 94% | 94% | 100% | 94% | 94% | 100% | 94% |
| OR Quart 2 | 0 | nd | >1.2 | 0.22 | >1.0 | 0.19 | 1.0 | >1.2 | 1.0 |
| p Value | na | nd | <0.89 | 0.26 | <1.0 | 0.21 | 1.0 | <0.89 | 1.0 |
| 95% CI of | na | nd | >0.058 | 0.017 | >0.055 | 0.015 | 0.048 | >0.067 | 0.048 |
| OR Quart2 | na | nd | na | 3.0 | na | 2.5 | 21 | na | 21 |
| OR Quart 3 | 1.0 | nd | >1.2 | 1.8 | >1.1 | 2.2 | 5.0 | >0 | 2.5 |
| p Value | 1.0 | nd | <0.89 | 0.59 | <0.94 | 0.45 | 0.24 | <na | 0.51 |
| 95% CI of | 0.045 | nd | >0.058 | 0.21 | >0.060 | 0.28 | 0.34 | >na | 0.16 |
| OR Quart3 | 22 | nd | na | 15 | na | 18 | 73 | na | 39 |
| OR Quart 4 | 1.0 | nd | >1.0 | 1.3 | >0 | 1.3 | 2.5 | >1.2 | 3.8 |
| p Value | 1.0 | nd | <1.0 | 0.78 | <na | 0.78 | 0.51 | <0.89 | 0.32 |
| 95% CI of | 0.045 | nd | >0.048 | 0.17 | >na | 0.17 | 0.16 | >0.067 | 0.27 |
| OR Quart4 | 22 | nd | na | 10 | na | 10 | 39 | na | 51 |

Fig. 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Hyaluronic acid**

| sCr or UO | 24hr prior to AKI stage | | 48hr prior to AKI stage | |
|---|---|---|---|---|
|  | Cohort 1 | Cohort 2 | Cohort 1 | Cohort 2 |
| Median | 343 | 2240 | 343 | 127 |
| Average | 969 | 2070 | 969 | 127 |
| Stdev | 1110 | 1280 | 1110 | 21.6 |
| p(t-test) |  | 0.065 |  | 0.29 |
| Min | 94.6 | 618 | 94.6 | 112 |
| Max | 3200 | 3200 | 3200 | 142 |
| n (Samp) | 43 | 4 | 43 | 2 |
| n (Patient) | 33 | 4 | 33 | 2 |

| sCr only | 24hr prior to AKI stage | | 48hr prior to AKI stage | |
|---|---|---|---|---|
|  | Cohort 1 | Cohort 2 | Cohort 1 | Cohort 2 |
| Median | nd | nd | 365 | 365 |
| Average | nd | nd | 1050 | 365 |
| Stdev | nd | nd | 1160 | 358 |
| p(t-test) | nd | nd |  | 0.41 |
| Min | nd | nd | 94.6 | 112 |
| Max | nd | nd | 3200 | 618 |
| n (Samp) | nd | nd | 47 | 2 |
| n (Patient) | nd | nd | 37 | 2 |

| UO only | 24hr prior to AKI stage | | 48hr prior to AKI stage | |
|---|---|---|---|---|
|  | Cohort 1 | Cohort 2 | Cohort 1 | Cohort 2 |
| Median | 343 | 2240 | nd | nd |
| Average | 945 | 2070 | nd | nd |
| Stdev | 1080 | 1280 | nd | nd |
| p(t-test) |  | 0.055 | nd | nd |
| Min | 140 | 618 | nd | nd |
| Max | 3200 | 3200 | nd | nd |
| n (Samp) | 41 | 4 | nd | nd |
| n (Patient) | 30 | 4 | nd | nd |

|  | 24hr prior to AKI stage | | | 48hr prior to AKI stage | | |
|---|---|---|---|---|---|---|
|  | sCr or UO | sCr only | UO only | sCr or UO | sCr only | UO only |
| ADC | 0.81 | nd | 0.81 | 0.047 | 0.32 | nd |
| SE | 0.14 | nd | 0.14 | 0.11 | 0.21 | nd |
| p | 0.025 | nd | 0.023 | 1.7E-5 | 0.40 | nd |
| nCohort 1 | 43 | nd | 41 | 43 | 47 | nd |
| nCohort 2 | 4 | nd | 4 | 2 | 2 | nd |
| Cutoff 1 | 1030 | nd | 1030 | 94.6 | 94.6 | nd |
| Sens 1 | 75% | nd | 75% | 100% | 100% | nd |
| Spec 1 | 74% | nd | 76% | 2% | 2% | nd |
| Cutoff 2 | 578 | nd | 578 | 94.6 | 94.6 | nd |
| Sens 2 | 100% | nd | 100% | 100% | 100% | nd |
| Spec 2 | 65% | nd | 66% | 2% | 2% | nd |
| Cutoff 3 | 578 | nd | 578 | 94.6 | 94.6 | nd |
| Sens 3 | 100% | nd | 100% | 100% | 100% | nd |
| Spec 3 | 65% | nd | 66% | 2% | 2% | nd |
| Cutoff 4 | 933 | nd | 898 | 933 | 1030 | nd |
| Sens 4 | 75% | nd | 75% | 0% | 0% | nd |
| Spec 4 | 72% | nd | 71% | 72% | 70% | nd |
| Cutoff 5 | 2150 | nd | 1860 | 2150 | 2530 | nd |
| Sens 5 | 50% | nd | 50% | 0% | 0% | nd |
| Spec 5 | 81% | nd | 80% | 81% | 81% | nd |
| Cutoff 6 | 3200 | nd | 3200 | 3200 | 3200 | nd |
| Sens 6 | 0% | nd | 0% | 0% | 0% | nd |
| Spec 6 | 100% | nd | 100% | 100% | 100% | nd |
| OR Quart 2 | >0 | nd | >0 | >0 | >1.2 | nd |

|  | 24hr prior to AKI stage | | | 48hr prior to AKI stage | | |
|---|---|---|---|---|---|---|
|  | sCr or UO | sCr only | UO only | sCr or UO | sCr only | UO only |
| p Value | <na | nd | <na | <na | <0.91 | nd |
| 95% CI of | >na | nd | >na | >na | >0.066 | nd |
| OR Quart2 | na | nd | na | na | na | nd |
| OR Quart 3 | >2.2 | nd | >2.4 | >0 | >0 | nd |
| p Value | <0.54 | nd | <0.49 | <na | <na | nd |
| 95% CI of | >0.17 | nd | >0.19 | >na | >na | nd |
| OR Quart3 | na | nd | na | na | na | nd |
| OR Quart 4 | >2.2 | nd | >2.2 | >2.7 | >1.2 | nd |
| p Value | <0.54 | nd | <0.54 | <0.45 | <0.91 | nd |
| 95% CI of | >0.17 | nd | >0.17 | >0.21 | >0.066 | nd |

(continued)

|  | 24hr prior to AKI stage | | | 48hr prior to AKI stage | | |
|---|---|---|---|---|---|---|
|  | sCr or UO | sCr only | UO only | sCr or UO | sCr only | UO only |
| OR Quart4 | na | nd | na | na | na | nd |

Fig. 7: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Hyaluronic acid**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 300 | 1390 | 300 | 1390 |
| Average | 794 | 1740 | 794 | 1740 |
| Stdev | 987 | 1330 | 987 | 1330 |
| p(t-test) |  | 0.17 |  | 0.17 |
| Min | 140 | 618 | 140 | 618 |
| Max | 3200 | 3200 | 3200 | 3200 |
| n (Samp) | 16 | 3 | 16 | 3 |
| n (Patient) | 16 | 3 | 16 | 3 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 305 | 1390 | 305 | 1390 |
| Average | 664 | 1740 | 664 | 1740 |
| Stdev | 799 | 1330 | 799 | 1330 |
| p(t-test) |  | 0.069 |  | 0.069 |
| Min | 140 | 618 | 140 | 618 |
| Max | 3200 | 3200 | 3200 | 3200 |
| n (Samp) | 16 | 3 | 16 | 3 |
| n (Patient) | 16 | 3 | 16 | 3 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| ADC | 0.82 | nd | 0.84 | 0.82 | nd | 0.84 |
| SE | 0.16 | nd | 0.15 | 0.16 | nd | 0.15 |
| p | 0.039 | nd | 0.021 | 0.039 | nd | 0.021 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| nCohort 1 | 16 | nd | 16 | 16 | nd | 16 |
| nCohort 2 | 3 | nd | 3 | 3 | nd | 3 |
| Cutoff 1 | 578 | nd | 578 | 578 | nd | 578 |
| Sens 1 | 100% | nd | 100% | 100% | nd | 100% |
| Spec 1 | 69% | nd | 69% | 69% | nd | 69% |
| Cutoff 2 | 578 | nd | 578 | 578 | nd | 578 |
| Sens 2 | 100% | nd | 100% | 100% | nd | 100% |
| Spec 2 | 69% | nd | 69% | 69% | nd | 69% |
| Cutoff 3 | 578 | nd | 578 | 578 | nd | 578 |
| Sens 3 | 100% | nd | 100% | 100% | nd | 100% |
| Spec 3 | 69% | nd | 69% | 69% | nd | 69% |
| Cutoff 4 | 933 | nd | 882 | 933 | nd | 882 |
| Sens 4 | 67% | nd | 67% | 67% | nd | 67% |
| Spec 4 | 75% | nd | 75% | 75% | nd | 75% |
| Cutoff 5 | 1030 | nd | 933 | 1030 | nd | 933 |
| Sens 5 | 67% | nd | 67% | 67% | nd | 67% |
| Spec 5 | 81% | nd | 81% | 81% | nd | 81% |
| Cutoff 6 | 2980 | nd | 1660 | 2980 | nd | 1660 |
| Sens 6 | 33% | nd | 33% | 33% | nd | 33% |
| Spec 6 | 94% | nd | 94% | 94% | nd | 94% |
| OR Quart 2 | >0 | nd | >0 | >0 | nd | >0 |
| p Value | <na | nd | <na | <na | nd | <na |
| 95% CI of | >na | nd | >na | >na | nd | >na |
| OR Quart2 | na | nd | na | na | nd | na |
| OR Quart 3 | >1.0 | nd | >1.0 | >1.0 | nd | >1.0 |
| p Value | <1.0 | nd | <1.0 | <1.0 | nd | <1.0 |
| 95% CI of OR Quart3 | >0.045 | nd | >0.045 | >0.045 | nd | >0.045 |
|  | na | nd | na | na | nd | na |
| OR Quart 4 | >2.7 | nd | >2.7 | >2.7 | nd | >2.7 |
| p Value | <0.50 | nd | <0.50 | <0.50 | nd | <0.50 |
| 95% CI of | >0.16 | nd | >0.16 | >0.16 | nd | >0.16 |
| OR Quart4 | na | nd | na | na | nd | na |

Fig. 8: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Hyaluronic acid**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1490 | 2590 | 1490 | 4100 | 1490 | 4060 |
| Average | 1920 | 3160 | 1920 | 3920 | 1920 | 3720 |
| Stdev | 1400 | 1990 | 1400 | 1720 | 1400 | 2110 |
| p(t-test) | | 9.1E-4 | | 2.5E-7 | | 0.0020 |
| Min | 77.8 | 390 | 77.8 | 783 | 77.8 | 379 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 6350 | 6400 | 6350 | 6400 | 6350 | 6190 |
| n (Samp) | 420 | 15 | 420 | 14 | 420 | 6 |
| n (Patient) | 132 | 15 | 132 | 14 | 132 | 6 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1640 | 2590 | 1640 | 4550 | 1640 | 2390 |
| Average | 2120 | 2950 | 2120 | 4240 | 2120 | 2690 |
| Stdev | 1570 | 2120 | 1570 | 955 | 1570 | 1550 |
| p(t-test) | | 0.24 | | 0.020 | | 0.53 |
| Min | 77.8 | 580 | 77.8 | 3170 | 77.8 | 1310 |
| Max | 6400 | 6400 | 6400 | 5000 | 6400 | 4360 |
| n (Samp) | 470 | 5 | 470 | 3 | 470 | 3 |
| n (Patient) | 145 | 5 | 145 | 3 | 145 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1530 | 2480 | 1530 | 4360 | 1530 | 4590 |
| Average | 1980 | 3480 | 1980 | 4090 | 1980 | 3990 |
| Stdev | 1430 | 2190 | 1430 | 1770 | 1430 | 2240 |
| p(t-test) | | 8.0E-4 | | 5.4E-8 | | 0.0021 |
| Min | 77.8 | 390 | 77.8 | 783 | 77.8 | 379 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 6350 | 6400 | 6350 | 6400 | 6350 | 6190 |
| n (Samp) | 380 | 11 | 380 | 15 | 380 | 5 |
| n (Patient) | 112 | 11 | 112 | 15 | 112 | 5 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.64 | 0.71 | 0.82 | 0.86 | 0.82 | 0.76 | 0.65 | 0.76 |
| SE | 0.077 | 0.13 | 0.089 | 0.070 | 0.14 | 0.067 | 0.12 | 0.17 | 0.13 |
| P | 0.013 | 0.29 | 0.018 | 6.4E-6 | 0.010 | 1.9E-6 | 0.027 | 0.40 | 0.039 |
| nCohort 1 | 420 | 470 | 380 | 420 | 470 | 380 | 420 | 470 | 380 |
| nCohort 2 | 15 | 5 | 11 | 14 | 3 | 15 | 6 | 3 | 5 |
| Cutoff 1 | 2420 | 2450 | 2420 | 3110 | 3110 | 3110 | 2380 | 1310 | 3510 |
| Sens 1 | 73% | 80% | 73% | 71% | 100% | 73% | 83% | 100% | 80% |
| Spec 1 | 72% | 67% | 70% | 80% | 76% | 79% | 71% | 41% | 83% |
| Cutoff 2 | 1710 | 2450 | 1710 | 2420 | 3110 | 2740 | 2380 | 1310 | 3510 |
| Sens 2 | 80% | 80% | 82% | 86% | 100% | 80% | 83% | 100% | 80% |
| Spec 2 | 56% | 67% | 53% | 72% | 76% | 75% | 71% | 41% | 83% |
| Cutoff 3 | 577 | 577 | 874 | 1330 | 3110 | 1330 | 373 | 1310 | 373 |
| Sens 3 | 93% | 100% | 91% | 93% | 100% | 93% | 100% | 100% | 100% |
| Spec 3 | 15% | 14% | 25% | 45% | 76% | 43% | 7% | 41% | 8% |
| Cutoff 4 | 2270 | 2620 | 2420 | 2270 | 2620 | 2420 | 2270 | 2620 | 2420 |
| Sens 4 | 73% | 40% | 73% | 86% | 100% | 87% | 83% | 33% | 80% |
| Spec 4 | 70% | 70% | 70% | 70% | 70% | 70% | 70% | 70% | 70% |
| Cutoff 5 | 3020 | 3520 | 3200 | 3020 | 3520 | 3200 | 3020 | 3520 | 3200 |
| Sens 5 | 40% | 20% | 45% | 71% | 67% | 67% | 67% | 33% | 80% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 4280 | 4690 | 4400 | 4280 | 4690 | 4400 | 4280 | 4690 | 4400 |
| Sens 6 | 33% | 20% | 36% | 50% | 33% | 47% | 50% | 0% | 60% |
| Spec 6 | 90% | 90% | 90% | 90% | 90% | 90% | 90% | 90% | 90% |
| OR Quart 2 | 0 | 0 | 0 | 0.99 | >0 | 0.99 | 0 | >1.0 | 0 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | na | na | na | 0.99 | <na | 0.99 | na | <1.00 | na |
| 95% CI of | na | na | na | 0.061 | >na | 0.061 | na | >0.062 | na |
| OR Quart2 | na | na | na | 16 | na | 16 | na | na | na |
| OR Quart 3 | 1.7 | 3.0 | 2.0 | 2.0 | >0 | 2.0 | 1.0 | >1.0 | 0 |
| p Value | 0.48 | 0.34 | 0.42 | 0.57 | <na | 0.57 | 1.0 | <1.00 | na |
| 95% CI of | 0.39 | 0.31 | 0.36 | 0.18 | >na | 0.18 | 0.062 | >0.062 | na |
| OR Quart3 | 7.2 | 30 | 11 | 23 | na | 22 | 16 | na | na |
| OR Quart 4 | 2.4 | 0.99 | 2.6 | 11 | >3.1 | 12 | 4.1 | >1.0 | 4.1 |
| p Value | 0.21 | 1.00 | 0.27 | 0.024 | <0.34 | 0.018 | 0.21 | <1.0 | 0.21 |
| 95% CI of | 0.60 | 0.061 | 0.48 | 1.4 | >0.31 | 1.5 | 0.45 | >0.062 | 0.45 |
| OR Quart4 | 9.5 | 16 | 13 | 86 | na | 96 | 37 | na | 37 |

Fig. 9: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

**Hyaluronic acid**

| sCr or UO | 24hr prior to AKI stage | |
|---|---|---|
| | Cohort 1 | Cohort 2 |
| Median | 331 | 1390 |
| Average | 949 | 1740 |
| Stdev | 1110 | 1330 |
| p(t-test) | | 0.24 |
| Min | 94.6 | 618 |
| Max | 3200 | 3200 |
| n (Samp) | 48 | 3 |
| n (Patient) | 37 | 3 |

| UO only | 24hr prior to AKI stage | |
|---|---|---|
| | Cohort 1 | Cohort 2 |
| Median | 338 | 1390 |

(continued)

| UO only | 24hr prior to AKI stage | |
|---|---|---|
| | Cohort 1 | Cohort 2 |
| Average | 957 | 1740 |
| Stdev | 1100 | 1330 |
| p(t-test) | | 0.25 |
| Min | 112 | 618 |
| Max | 3200 | 3200 |

| UO only | 24hr prior to AKI stage | |
|---|---|---|
| | Cohort 1 | Cohort 2 |
| n (Samp) | 44 | 3 |
| n (Patient) | 33 | 3 |

| | 24hr prior to AKI stage | | |
|---|---|---|---|
| | sCr or UO | sCr only | UO only |
| AUC | 0.79 | nd | 0.78 |
| SE | 0.16 | nd | 0.16 |
| p | 0.072 | nd | 0.078 |
| nCohort 1 | 48 | nd | 44 |
| nCohort 2 | 3 | nd | 3 |
| Cutoff 1 | 578 | nd | 578 |
| Sens 1 | 100% | nd | 100% |
| Spec 1 | 67% | nd | 66% |
| Cutoff 2 | 578 | nd | 578 |
| Sens 2 | 100% | nd | 100% |
| Spec 2 | 67% | nd | 66% |
| Cutoff 3 | 578 | nd | 578 |
| Sens 3 | 100% | nd | 100% |
| Spec 3 | 67% | nd | 66% |
| Cutoff 4 | 898 | nd | 898 |
| Sens 4 | 67% | nd | 67% |
| Spec 4 | 71% | nd | 70% |
| Cutoff 5 | 2150 | nd | 2150 |
| Sens 5 | 33% | nd | 33% |
| Spec 5 | 81% | nd | 82% |
| Cutoff 6 | 3200 | nd | 3200 |

(continued)

| | 24hr prior to AKI stage | | |
|---|---|---|---|
| | sCr or UO | sCr only | UO only |
| Sens 6 | 0% | nd | 0% |
| Spec 6 | 100% | nd | 100% |
| OR | >0 | nd | >0 |
| Quart 2 | <na | nd | <na |
| p Value | >na | nd | >na |
| 95% CI of OR Quart2 | na | nd | na |
| OR | >2.2 | nd | >2.2 |
| Quart 3 | <0.55 | nd | <0.54 |
| p Value | >0.17 | nd | >0.17 |

| | 24hr prior to AKI stage | | |
|---|---|---|---|
| | sCr or UO | sCr only | UO only |
| 95% CI of OR Quart3 | na | nd | na |
| OR | >1.0 | nd | >1.0 |
| Quart 4 | <1.0 | nd | <1.0 |
| p Value | >0.056 | nd | >0.055 |
| 95% CI of OR Quart4 | na | nd | na |

Fig. 10: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

**Hyaluronic acid**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1190 | 2850 | 1350 | 2890 | 1250 | 2930 |
| Average | 1520 | 2970 | 1740 | 3010 | 1610 | 3070 |
| Stdev | 1180 | 1730 | 1380 | 1780 | 1190 | 1770 |
| p(t-test) | | 1.9E-18 | | 9.1E-5 | | 5.7E-15 |
| Min | 69.2 | 197 | 69.2 | 197 | 77.8 | 452 |
| Max | 6300 | 6400 | 6400 | 6390 | 5430 | 6400 |
| n (Samp) | 350 | 84 | 410 | 20 | 267 | 71 |
| n (Patient) | 350 | 84 | 410 | 20 | 267 | 71 |

|  | At Enrollment | | |
|---|---|---|---|
|  | sCr or UO | sCr only | UO only |
| AUC | 0.76 | 0.71 | 0.75 |
| SE | 0.032 | 0.066 | 0.036 |
| P | 1.1E-15 | 0.0013 | 1.5E-12 |
| nCohort 1 | 350 | 410 | 267 |
| nCohort 2 | 84 | 20 | 71 |
| Cutoff 1 | 1680 | 2700 | 1680 |
| Sens 1 | 70% | 70% | 70% |
| Spec 1 | 67% | 80% | 63% |
| Cutoff 2 | 1400 | 1450 | 1440 |
| Sens 2 | 81% | 80% | 80% |
| Spec 2 | 58% | 54% | 57% |

|  | At Enrollment | | |
|---|---|---|---|
|  | sCr or UO | sCr only | UO only |
| Cutoff 3 | 752 | 555 | 781 |
| Sens 3 | 90% | 90% | 90% |
| Spec 3 | 30% | 19% | 27% |
| Cutoff 4 | 1830 | 2030 | 1980 |
| Sens 4 | 69% | 75% | 65% |
| Spec 4 | 70% | 70% | 70% |
| Cutoff 5 | 2210 | 2740 | 2410 |
| Sens 5 | 62% | 60% | 58% |
| Spec 5 | 80% | 80% | 80% |
| Cutoff 6 | 3190 | 3790 | 3330 |
| Sens 6 | 40% | 30% | 39% |
| Spec 6 | 90% | 90% | 90% |
| OR Quart 2 | 1.3 | 0 | 1.1 |
| p Value | 0.62 | na | 0.82 |
| 95% CI of | 0.47 | na | 0.41 |
| OR Quart2 | 3.6 | na | 3.1 |
| OR Quart 3 | 3.3 | 0.49 | 2.1 |
| p Value | 0.010 | 0.42 | 0.12 |
| 95% CI of | 1.3 | 0.088 | 0.82 |
| OR Quart3 | 8.1 | 2.7 | 5.2 |
| OR Quart 4 | 11 | 3.8 | 8.1 |
| p Value | 2.5E-8 | 0.021 | 1.3E-6 |
| 95% CI of | 4.8 | 1.2 | 3.5 |

(continued)

|  | At Enrollment | | |
|---|---|---|---|
|  | sCr or UO | sCr only | UO only |
| OR Quart4 | 27 | 12 | 19 |

Fig. 11: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

**Hyaluronic acid**

|  | sCr or UO | | UO only | |
|---|---|---|---|---|
|  | Cohort 1 | Cohort 2 | Cohort 1 | Cohort 2 |
| Median | 265 | 519 | 264 | 519 |
| Average | 722 | 1070 | 711 | 1070 |
| Stdev | 921 | 1280 | 902 | 1280 |
| p(t-test) |  | 0.33 |  | 0.32 |
| Min | 86.8 | 63.6 | 86.8 | 63.6 |
| Max | 3200 | 3200 | 3200 | 3200 |
| n (Samp) | 50 | 9 | 43 | 9 |
| n (Patient) | 50 | 9 | 43 | 9 |

|  | At Enrollment | |
|---|---|---|
|  | sCr or UO | UO only |
| AUC | 0.52 | 0.51 |
| SE | 0.11 | 0.11 |
| P | 0.84 | 0.94 |
| nCohort 1 | 50 | 43 |
| nCohort 2 | 9 | 9 |
| Cutoff 1 | 140 | 140 |
| Sens 1 | 78% | 78% |
| Spec 1 | 16% | 12% |
| Cutoff 2 | 112 | 102 |
| Sens 2 | 89% | 89% |
| Spec 2 | 8% | 5% |
| Cutoff 3 | 0 | 0 |
| Sens 3 | 100% | 100% |
| Spec 3 | 0% | 0% |
| Cutoff 4 | 476 | 515 |
| Sens 4 | 56% | 56% |

(continued)

| | At Enrollment | |
|---|---|---|
| | sCr or UO | UO only |
| Spec 4 | 70% | 72% |
| Cutoff 5 | 1030 | 1030 |
| Sens 5 | 33% | 33% |
| Spec 5 | 80% | 81% |
| Cutoff 6 | 2190 | 2190 |
| Sens 6 | 22% | 22% |
| Spec 6 | 90% | 91% |
| OR Quart 2 | 0.26 | 0.28 |
| p Value | 0.27 | 0.30 |
| 95% CI of | 0.024 | 0.025 |
| OR Quart2 | 2.9 | 3.1 |
| OR Quart 3 | 0.56 | 0.61 |
| p Value | 0.57 | 0.62 |
| 95% CI of | 0.079 | 0.083 |
| OR Quart3 | 4.0 | 4.4 |
| OR Quart 4 | 0.92 | 1.0 |
| p Value | 0.92 | 1.0 |
| 95% CI of | 0.15 | 0.16 |
| OR Quart4 | 5.5 | 6.2 |

The examples provided herein are representative of preferred embodiments, are exemplary, and are not intended as limitations on the scope of the invention. Modifications therein and other uses will occur to those skilled in the art.

**Claims**

1. A method for evaluating renal status in a sepsis patient, comprising:

performing one or more assays configured to detect hyaluronic acid in a urine sample obtained from the sepsis patient to provide an assay result; and
correlating the assay result to the renal status of the sepsis patient, wherein either:

(i) the sepsis patient is in RIFLE stage 0 or R, and said correlating step comprises assigning a likelihood that the patient will develop a RIFLE I or RIFLE F acute kidney injury within 72 hours of the time the sample is obtained from the patient; or
(ii) the sepsis patient is in RIFLE stage I, and said correlating step comprises assigning a likelihood that the patient will develop a RIFLE F acute kidney injury within 72 hours of the time the sample is obtained from the patient.

2. A method according to claim 1, wherein said correlation step comprises assigning a likelihood of a clinical outcome related to a renal injury suffered by the sepsis patient.

3. A method according to claim 1, wherein the correlating step comprises assigning a likelihood that the patient will develop the RIFLE I or RIFLE F acute kidney injury within 48 hours or 24 hours of the time the sample is obtained from the patient.

4. A method according to one of claims 1-2, wherein the sepsis patient is a severe sepsis patient, or

> wherein the sepsis patient is a septic shock patient, or
> wherein said correlating step comprises assessing whether or not renal function is improving or worsening in a sepsis patient who has suffered from ARF based on the assay result.

5. A method according to one of claims 1-2, wherein said correlating step comprises diagnosing the occurrence or nonoccurrence of a need for renal transplantation in said sepsis patient, or

> wherein said correlating step comprises diagnosing the occurrence or nonoccurrence of a need for renal replacement therapy in said sepsis patient, or
> wherein said correlating step comprises predicting a decreased or increased risk that the sepsis patient will require renal replacement therapy, or
> wherein said correlating step comprises predicting a decreased or increased risk that the sepsis patient will require renal transplantation.

6. A method according to one of claims 1-2, wherein the sepsis patient is in RIFLE stage 0 or R.

7. A method according to claim 6, wherein the sepsis patient is in RIFLE stage 0; or

> wherein the sepsis patient is in RIFLE stage 0, and said correlating step comprises assigning a likelihood that the sepsis patient will reach RIFLE stage I within 72 hours, within 48 hours, or within 24 hours; or
> wherein the sepsis patient is in RIFLE stage 0, and said correlating step comprises assigning a likelihood that the sepsis patient will reach RIFLE stage I or F within 72 hours, within 48 hours, or within 24 hours; or
> wherein the sepsis patient is in RIFLE stage 0, and said correlating step comprises assigning a likelihood that the sepsis patient will reach RIFLE stage F within 72 hours, within 48 hours, or within 24 hours; or
> wherein the sepsis patient is in RIFLE stage 0 or R, and said correlating step comprises assigning a likelihood that the sepsis patient will reach RIFLE stage I or F within 72 hours, within 48 hours, or within 24 hours; or
> wherein the sepsis patient is in RIFLE stage 0 or R, and said correlating step comprises assigning a likelihood that the sepsis patient will reach RIFLE stage F within 72 hours, within 48 hours, or within 24 hours.

8. A method according to claim 6, wherein the sepsis patient is in RIFLE stage R; or

> wherein the sepsis patient is in RIFLE stage R, and said correlating step comprises assigning a likelihood that the sepsis patient will reach RIFLE stage I within 72 hours, within 48 hours, or within 24 hours; or
> wherein the sepsis patient is in RIFLE stage R, and said correlating step comprises assigning a likelihood that the sepsis patient will reach RIFLE stage F within 72 hours within 48 hours, or within 24 hours.

9. A method according to one of claims 1-2, wherein the sepsis patient is in RIFLE stage 0, R, or I, and said correlating step comprises assigning a likelihood that the sepsis patient will reach RIFLE stage F within 72 hours, within 48 hours, or within 24 hours, or
wherein the sepsis patient is in RIFLE stage I, and said correlating step comprises assigning a likelihood that the sepsis patient will reach RIFLE stage F within 72 hours, within 48 hours, or within 24 hours.

10. A method according to one of claims 1-2, wherein the sepsis patient has not experienced a 1.5-fold or greater increase in serum creatinine over a baseline value determined prior to the time at which the body fluid sample is obtained, or

> wherein the sepsis patient has a urine output of at least 0.5 ml/kg/hr over the 6 hours preceding the time at which the body fluid sample is obtained, or
> wherein the sepsis patient has not experienced an increase of 0.3 mg/dL or greater in serum creatinine over a baseline value determined prior to the time at which the body fluid sample is obtained, or
> wherein the sepsis patient (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained.

11. A method according to one of claims 1-2, wherein the sepsis patient has not experienced a 2-fold or greater increase in serum creatinine over a baseline value determined prior to the time at which the body fluid sample is obtained, or

wherein the sepsis patient has a urine output of at least 0.5 ml/kg/hr over the 12 hours preceding the time at which the body fluid sample is obtained, or
wherein the sepsis patient (i) has not experienced a 2-fold or greater increase in serum creatinine over a baseline value determined prior to the time at which the body fluid sample is obtained, and (ii) has a urine output of at least 0.5 ml/kg/hr over the 12 hours preceding the time at which the body fluid sample is obtained.

12. A method according to one of claims 1-2, wherein the sepsis patient has not experienced a 3-fold or greater increase in serum creatinine over a baseline value determined prior to the time at which the body fluid sample is obtained, or

wherein the sepsis patient has a urine output of at least 0.3 ml/kg/hr over the 24 hours preceding the time at which the body fluid sample is obtained, and has not experienced anuria over the 12 hours preceding the time at which the body fluid sample is obtained, or
wherein the sepsis patient (i) has not experienced a 3-fold or greater increase in serum creatinine over a baseline value determined prior to the time at which the body fluid sample is obtained, and (ii) has a urine output of at least 0.3 ml/kg/hr over the 24 hours preceding the time at which the body fluid sample is obtained, and has not experienced anuria over the 12 hours preceding the time at which the body fluid sample is obtained.

13. A method according to one of claims 1-2, wherein said correlating step comprises assigning a likelihood that within 72 hours the sepsis patient will (i) experience a 2-fold or greater increase in serum creatinine and (ii) have a urine output of less than 0.5 ml/kg/hr over a 12 hour period, or

wherein said correlating step comprises assigning a likelihood that within 48 hours the sepsis patient will (i) experience a 2-fold or greater increase in serum creatinine, and (ii) have a urine output of less than 0.5 ml/kg/hr over a 12 hour period, or
wherein said correlating step comprises assigning a likelihood that within 24 hours the sepsis patient will (i) experience a 2-fold or greater increase in serum creatinine, and (ii) have a urine output of less than 0.5 ml/kg/hr over a 12 hour period, or
wherein said correlating step comprises assigning a likelihood that within 72 hours the sepsis patient will experience a 2-fold or greater increase in serum creatinine, or
wherein said correlating step comprises assigning a likelihood that within 72 hours the sepsis patient will have a urine output of less than 0.5 ml/kg/hr over a 12 hour period, or
wherein said correlating step comprises assigning a likelihood that within 48 hours the sepsis patient will experience a 2-fold or greater increase in serum creatinine, or
wherein said correlating step comprises assigning a likelihood that within 48 hours the sepsis patient will have a urine output of less than 0.5 ml/kg/hr over a 12 hour period, or
wherein said correlating step comprises assigning a likelihood that within 24 hours the sepsis patient will experience a 2-fold or greater increase in serum creatinine, or
wherein said correlating step comprises assigning a likelihood that within 24 hours the sepsis patient will have a urine output of less than 0.5 ml/kg/hr over a 12 hour period, or
wherein said correlating step comprises assigning a likelihood that within 72 hours the sepsis patient will (i) experience a 3-fold or greater increase in serum creatinine, and (ii) have a urine output of less than 0.3 ml/kg/hr over a 24 hour period or anuria over a 12 hour period.

14. A method according to one of claims 1-2, wherein said correlating step comprises assigning a likelihood that within 48 hours the sepsis patient will (i) experience a 3-fold or greater increase in serum creatinine, and (ii) have a urine output of less than 0.3 ml/kg/hr over a 24 hour period or anuria over a 12 hour period, or

wherein said correlating step comprises assigning a likelihood that within 24 hours the sepsis patient will (i) experience a 3-fold or greater increase in serum creatinine, and (ii) have a urine output of less than 0.3 ml/kg/hr over a 24 hour period or anuria over a 12 hour period, or
wherein said correlating step comprises assigning a likelihood that within 72 hours the sepsis patient will experience a 3-fold or greater increase in serum creatinine, or
wherein said correlating step comprises assigning a likelihood that within 72 hours the sepsis patient will have a urine output of less than 0.3 ml/kg/hr over a 24 hour period or anuria over a 12 hour period, or
wherein said correlating step comprises assigning a likelihood that within 48 hours the sepsis patient will experience a 3-fold or greater increase in serum creatinine, or
wherein said correlating step comprises assigning a likelihood that within 48 hours the sepsis patient will have a urine output of less than 0.3 ml/kg/hr over a 24 hour period or anuria over a 12 hour period, or
wherein said correlating step comprises assigning a likelihood that within 24 hours the sepsis patient will experience a 3-fold or greater increase in serum creatinine, or
wherein said correlating step comprises assigning a likelihood that within 24 hours the sepsis patient will have a urine output of less than 0.3 ml/kg/hr over a 24 hour period or anuria over a 12 hour period.

**Patentansprüche**

1. Ein Verfahren zur Bewertung des Nierenstatus bei einem Sepsispatienten, das Folgendes beinhaltet:

   Durchführen eines oder mehrerer Assays, die dazu ausgelegt sind, Hyaluronsäure in einer Urinprobe nachzuweisen, die von dem Sepsispatienten erhalten wird, um ein Assayergebnis bereitzustellen; und
   Korrelieren des Assayergebnisses mit dem Nierenstatus des Sepsispatienten, wobei entweder:

   (i) der Sepsispatient im RIFLE-Stadium 0 oder R ist und der genannte Korrelationsschritt das Zuweisen einer Wahrscheinlichkeit beinhaltet, dass bei dem Patienten innerhalb von 72 Stunden ab dem Zeitpunkt, zu dem die Probe von dem Patienten erhalten wird, eine RIFLE I oder RIFLE F akute Nierenschädigung eintreten wird, beinhaltet; oder
   (ii) der Sepsispatient im RIFLE-Stadium I ist und der genannte Korrelationsschritt das Zuweisen einer Wahrscheinlichkeit beinhaltet, dass bei dem Patienten innerhalb von 72 Stunden ab dem Zeitpunkt, zu dem die Probe von dem Patienten erhalten wird, eine RIFLE F akute Nierenschädigung eintreten wird.

2. Verfahren nach Anspruch 1, wobei der genannte Korrelationsschritt das Zuweisen einer Wahrscheinlichkeit eines klinischen Befunds in Bezug auf eine von dem Sepsispatienten erlittene Nierenschädigung beinhaltet.

3. Verfahren nach Anspruch 1, wobei der Korrelationsschritt das Zuweisen einer Wahrscheinlichkeit beinhaltet, dass die RIFLE I oder RIFLE F akute Nierenschädigung, innerhalb von 48 Stunden oder 24 Stunden ab dem Zeitpunkt, zu dem die Probe von dem Patienten erhalten wird, bei dem Patienten eintreten wird.

4. Verfahren nach einem der Ansprüche 1-2, wobei der Sepsispatient ein Patient mit einer schweren Sepsis ist, oder

   wobei der Sepsispatient ein Patient mit einem septischen Schock ist, oder
   wobei der genannte Korrelationsschritt eine Beurteilung, ob sich bei einem Sepsispatienten, der eine ARF erlitten hat, die Nierenfunktion verbessert oder verschlechtert, auf der Grundlage des Assayergebnisses beinhaltet.

5. Verfahren nach einem der Ansprüche 1-2, wobei der genannte Korrelationsschritt das Diagnostizieren des Eintritts oder Nichteintritts eines Nierentransplantationsbedarfs bei dem genannten Sepsispatienten beinhaltet oder

   wobei der genannte Korrelationsschritt das Diagnostizieren des Eintritts oder Nichteintritts eines Nierenersatztherapiebedarfs bei dem genannten Sepsispatienten beinhaltet oder
   wobei der genannte Korrelationsschritt das Vorhersagen eines verringerten oder erhöhten Risikos, dass bei dem Sepsispatienten eine Nierenersatztherapie erforderlich sein wird, beinhaltet oder
   wobei der genannte Korrelationsschritt das Vorhersagen eines verringerten oder erhöhten Risikos, dass bei dem Sepsispatienten eine Nierentransplantation erforderlich sein wird, beinhaltet.

6. Verfahren nach einem der Ansprüche 1-2, wobei der Sepsispatient im RIFLE-Stadium 0 oder R ist.

7. Verfahren nach Anspruch 6, wobei der Sepsispatient im RIFLE-Stadium 0 ist; oder

   wobei der Sepsispatient im RIFLE-Stadium 0 ist und der genannte Korrelationsschritt das Zuweisen einer Wahrscheinlichkeit beinhaltet, dass der Sepsispatient das RIFLE-Stadium I innerhalb von 72 Stunden, innerhalb von 48 Stunden oder innerhalb von 24 Stunden erreichen wird; oder

wobei der Sepsispatient im RIFLE-Stadium 0 ist und der genannte Korrelationsschritt das Zuweisen einer Wahrscheinlichkeit beinhaltet, dass der Sepsispatient das RIFLE-Stadium I oder F innerhalb von 72 Stunden, innerhalb von 48 Stunden oder innerhalb von 24 Stunden erreichen wird; oder

wobei der Sepsispatient im RIFLE-Stadium 0 ist und der genannte Korrelationsschritt das Zuweisen einer Wahrscheinlichkeit beinhaltet, dass der Sepsispatient das RIFLE-Stadium F innerhalb von 72 Stunden, innerhalb von 48 Stunden oder innerhalb von 24 Stunden erreichen wird; oder

wobei der Sepsispatient im RIFLE-Stadium 0 oder R ist und der genannte Korrelationsschritt das Zuweisen einer Wahrscheinlichkeit beinhaltet, dass der Sepsispatient das RIFLE-Stadium I oder F innerhalb von 72 Stunden, innerhalb von 48 Stunden oder innerhalb von 24 Stunden erreichen wird; oder

wobei der Sepsispatient im RIFLE-Stadium 0 oder R ist und der genannte Korrelationsschritt das Zuweisen einer Wahrscheinlichkeit beinhaltet, dass der Sepsispatient das RIFLE-Stadium F innerhalb von 72 Stunden, innerhalb von 48 Stunden oder innerhalb von 24 Stunden erreichen wird.

8. Verfahren nach Anspruch 6, wobei der Sepsispatient im RIFLE-Stadium R ist; oder

wobei der Sepsispatient im RIFLE-Stadium R ist und der genannte Korrelationsschritt das Zuweisen einer Wahrscheinlichkeit beinhaltet, dass der Sepsispatient das RIFLE-Stadium I innerhalb von 72 Stunden, innerhalb von 48 Stunden oder innerhalb von 24 Stunden erreichen wird; oder

wobei der Sepsispatient im RIFLE-Stadium R ist und der genannte Korrelationsschritt das Zuweisen einer Wahrscheinlichkeit beinhaltet, dass der Sepsispatient das RIFLE-Stadium F innerhalb von 72 Stunden, innerhalb von 48 Stunden oder innerhalb von 24 Stunden erreichen wird.

9. Verfahren nach einem der Ansprüche 1-2, wobei der Sepsispatient im RIFLE-Stadium 0, R oder I ist und der genannte Korrelationsschritt das Zuweisen einer Wahrscheinlichkeit beinhaltet, dass der Sepsispatient das RIFLE-Stadium F innerhalb von 72 Stunden, innerhalb von 48 Stunden oder innerhalb von 24 Stunden erreichen wird, oder

wobei der Sepsispatient im RIFLE-Stadium I ist und der genannte Korrelationsschritt das Zuweisen einer Wahrscheinlichkeit beinhaltet, dass der Sepsispatient das RIFLE-Stadium F innerhalb von 72 Stunden, innerhalb von 48 Stunden oder innerhalb von 24 Stunden erreichen wird.

10. Verfahren nach einem der Ansprüche 1-2, wobei der Sepsispatient keinen 1,5-fachen oder höheren Anstieg des Serumkreatinins gegenüber einem Ausgangswert, der vor dem Zeitpunkt bestimmt wurde, zu dem die Körperflüssigkeitsprobe erhalten wird, erfahren hat, oder

wobei der Sepsispatient eine Urinausscheidung von mindestens 0,5 ml/kg/h über die 6 Stunden, die dem Zeitpunkt vorausgehen, zu dem die Körperflüssigkeitsprobe erhalten wird, aufweist, oder

wobei der Sepsispatient keinen Anstieg des Serumkreatinins von 0,3 mg/dl oder mehr gegenüber einem Ausgangswert, der vor dem Zeitpunkt bestimmt wurde, zu dem die Körperflüssigkeitsprobe erhalten wird, erfahren hat, oder

wobei der Sepsispatient (i) keinen 1,5-fachen oder höheren Anstieg des Serumkreatinins gegenüber einem Ausgangswert, der vor dem Zeitpunkt bestimmt wurde, zu dem die Körperflüssigkeitsprobe erhalten wird, erfahren hat, (ii) eine Urinausscheidung von mindestens 0,5 ml/kg/h über die 6 Stunden, die dem Zeitpunkt vorausgehen, zu dem die Körperflüssigkeitsprobe erhalten wird, aufweist und (iii) keinen Anstieg des Serumkreatinins von 0,3 mg/dl oder mehr gegenüber einem Ausgangswert, der vor dem Zeitpunkt, zu dem die Körperflüssigkeitsprobe erhalten wird, erfahren hat.

11. Verfahren nach einem der Ansprüche 1-2, wobei der Sepsispatient keinen 2-fachen oder höheren Anstieg des Serumkreatinins gegenüber einem Ausgangswert, der vor dem Zeitpunkt bestimmt wurde, zu dem die Körperflüssigkeitsprobe erhalten wird, erfahren hat, oder

wobei der Sepsispatient eine Urinausscheidung von mindestens 0,5 ml/kg/h über die 12 Stunden, die dem Zeitpunkt vorausgehen, zu dem die Körperflüssigkeitsprobe erhalten wird, aufweist oder

wobei der Sepsispatient (i) keinen 2-fachen oder höheren Anstieg des Serumkreatinins gegenüber einem Ausgangswert, der vor dem Zeitpunkt bestimmt wurde, zu dem die Körperflüssigkeitsprobe erhalten wird, erfahren hat und (ii) eine Urinausscheidung von mindestens 0,5 ml/kg/h über die 12 Stunden, die dem Zeitpunkt vorausgehen, zu dem die Körperflüssigkeitsprobe erhalten wird, aufweist.

12. Verfahren nach einem der Ansprüche 1-2, wobei der Sepsispatient keinen 3-fachen oder höheren Anstieg des Serumkreatinins gegenüber einem Ausgangswert, der vor dem Zeitpunkt bestimmt wurde, zu dem die Körperflüssigkeitsprobe erhalten wird, erfahren hat oder

wobei der Sepsispatient eine Urinausscheidung von mindestens 0,3 ml/kg/h über die 24 Stunden, die dem Zeitpunkt vorausgehen, zu dem die Körperflüssigkeitsprobe erhalten wird, aufweist und keine Anurie über die 12 Stunden, die dem Zeitpunkt vorausgehen, zu dem die Körperflüssigkeitsprobe erhalten wird, erfahren hat, oder

wobei der Sepsispatient (i) keinen 3-fachen oder höheren Anstieg des Serumkreatinins gegenüber einem Ausgangswert, der vor dem Zeitpunkt bestimmt wurde, zu dem die Körperflüssigkeitsprobe erhalten wird, erfahren hat und (ii) eine Urinausscheidung von mindestens 0,3 ml/kg/h über die 24 Stunden, die dem Zeitpunkt vorausgehen, zu dem die Körperflüssigkeitsprobe erhalten wird, aufweist und keine Anurie über die 12 Stunden, die dem Zeitpunkt vorausgehen, zu dem die Körperflüssigkeitsprobe erhalten wird, erfahren hat.

13. Verfahren nach einem der Ansprüche 1-2, wobei der genannte Korrelationsschritt das Zuweisen einer Wahrscheinlichkeit beinhaltet, dass der Sepsispatient innerhalb von 72 Stunden (i) einen 2-fachen oder höheren Anstieg des Serumkreatinins erfahren wird und (ii) eine Urinausscheidung von weniger als 0,5 ml/kg/h über einen 12-stündigen Zeitraum aufweisen wird, oder

wobei der genannte Korrelationsschritt das Zuweisen einer Wahrscheinlichkeit beinhaltet, dass der Sepsispatient innerhalb von 48 Stunden (i) einen 2-fachen oder höheren Anstieg des Serumkreatinins erfahren wird und (ii) eine Urinausscheidung von weniger als 0,5 ml/kg/h über einen 12-stündigen Zeitraum aufweisen wird, oder

wobei der genannte Korrelationsschritt das Zuweisen einer Wahrscheinlichkeit beinhaltet, dass der Sepsispatient innerhalb von 24 Stunden (i) einen 2-fachen oder höheren Anstieg des Serumkreatinins erfahren wird und (ii) eine Urinausscheidung von weniger als 0,5 ml/kg/h über einen 12-stündigen Zeitraum aufweisen wird, oder

wobei der genannte Korrelationsschritt das Zuweisen einer Wahrscheinlichkeit beinhaltet, dass der Sepsispatient innerhalb von 72 Stunden einen 2-fachen oder höheren Anstieg des Serumkreatinins erfahren wird, oder

wobei der genannte Korrelationsschritt das Zuweisen einer Wahrscheinlichkeit beinhaltet, dass der Sepsispatient innerhalb von 72 Stunden eine Urinausscheidung von weniger als 0,5 ml/kg/h über einen 12-stündigen Zeitraum aufweisen wird, oder

wobei der genannte Korrelationsschritt das Zuweisen einer Wahrscheinlichkeit beinhaltet, dass der Sepsispatient innerhalb von 48 Stunden einen 2-fachen oder höheren Anstieg des Serumkreatinins erfahren wird, oder

wobei der genannte Korrelationsschritt das Zuweisen einer Wahrscheinlichkeit beinhaltet, dass der Sepsispatient innerhalb von 48 Stunden eine Urinausscheidung von weniger als 0,5 ml/kg/h über einen 12-stündigen Zeitraum aufweisen wird, oder

wobei der genannte Korrelationsschritt das Zuweisen einer Wahrscheinlichkeit beinhaltet, dass der Sepsispatient innerhalb von 24 Stunden einen 2-fachen oder höheren Anstieg des Serumkreatinins erfahren wird, oder

wobei der genannte Korrelationsschritt das Zuweisen einer Wahrscheinlichkeit beinhaltet, dass der Sepsispatient innerhalb von 24 Stunden eine Urinausscheidung von weniger als 0,5 ml/kg/h über einen 12-stündigen Zeitraum aufweisen wird, oder

wobei der genannte Korrelationsschritt das Zuweisen einer Wahrscheinlichkeit beinhaltet, dass der Sepsispatient innerhalb von 72 Stunden (i) einen 3-fachen oder höheren Anstieg des Serumkreatinins erfahren wird und (ii) eine Urinausscheidung von weniger als 0,3 ml/kg/h über einen 24-stündigen Zeitraum oder eine Anurie über einen 12-stündigen Zeitraum aufweisen wird.

14. Verfahren nach einem der Ansprüche 1-2, wobei der genannte Korrelationsschritt das Zuweisen einer Wahrscheinlichkeit beinhaltet, dass der Sepsispatient innerhalb von 48 Stunden (i) einen 3-fachen oder höheren Anstieg des Serumkreatinins erfahren wird und (ii) eine Urinausscheidung von weniger als 0,3 ml/kg/h über einen 24-stündigen Zeitraum oder eine Anurie über einen 12-stündigen Zeitraum aufweisen wird, oder

wobei der genannte Korrelationsschritt das Zuweisen einer Wahrscheinlichkeit beinhaltet, dass der Sepsispatient innerhalb von 24 Stunden (i) einen 3-fachen oder höheren Anstieg des Serumkreatinins erfahren wird und (ii) eine Urinausscheidung von weniger als 0,3 ml/kg/h über einen 24-stündigen Zeitraum oder eine Anurie über einen 12-stündigen Zeitraum aufweisen wird, oder

wobei der genannte Korrelationsschritt das Zuweisen einer Wahrscheinlichkeit beinhaltet, dass der Sepsispatient innerhalb von 72 Stunden einen 3-fachen oder höheren Anstieg des Serumkreatinins erfahren wird, oder

wobei der genannte Korrelationsschritt das Zuweisen einer Wahrscheinlichkeit beinhaltet, dass der Sepsispatient innerhalb von 72 Stunden eine Urinausscheidung von weniger als 0,3 ml/kg/h über einen 24-stündigen Zeitraum oder eine Anurie über einen 12-stündigen Zeitraum aufweisen wird, oder

wobei der genannte Korrelationsschritt das Zuweisen einer Wahrscheinlichkeit beinhaltet, dass der Sepsispatient innerhalb von 48 Stunden einen 3-fachen oder höheren Anstieg des Serumkreatinins erfahren wird, oder wobei der genannte Korrelationsschritt das Zuweisen einer Wahrscheinlichkeit beinhaltet, dass der Sepsispatient innerhalb von 48 Stunden eine Urinausscheidung von weniger als 0,3 ml/kg/h über einen 24-stündigen Zeitraum oder eine Anurie über einen 12-stündigen Zeitraum aufweisen wird, oder

wobei der genannte Korrelationsschritt das Zuweisen einer Wahrscheinlichkeit beinhaltet, dass der Sepsispatient innerhalb von 24 Stunden einen 3-fachen oder höheren Anstieg des Serumkreatinins erfahren wird, oder wobei der genannte Korrelationsschritt das Zuweisen einer Wahrscheinlichkeit beinhaltet, dass der Sepsispatient innerhalb von 24 Stunden eine Urinausscheidung von weniger als 0,3 ml/kg/h über einen 24-stündigen Zeitraum oder eine Anurie über einen 12-stündigen Zeitraum aufweisen wird.

**Revendications**

1. Procédé d'évaluation de l'état rénal chez un patient présentant un sepsis, comprenant :

la réalisation d'une ou plusieurs analyses configurées pour détecter de l'acide hyaluronique dans un échantillon d'urine prélevé sur le patient présentant un sepsis pour fournir un résultat d'analyse ; et
la corrélation du résultat d'analyse à l'état rénal du patient présentant un sepsis, dans lequel, soit :

(i) le patient présentant un sepsis est dans la classe 0 ou R de RIFLE, et ladite étape de corrélation comprend l'assignation d'une probabilité que le patient développera une lésion rénale aiguë dans la classe I ou F de RIFLE dans les 72 heures suivant le moment où l'échantillon est prélevé sur le patient ; soit
(ii) le patient présentant un sepsis est dans la classe I de RIFLE, et ladite étape de corrélation comprend l'assignation d'une probabilité que le patient développera une lésion rénale aiguë dans la classe F de RIFLE dans les 72 heures suivant le moment où l'échantillon est prélevé sur le patient.

2. Procédé selon la revendication 1, dans lequel ladite étape de corrélation comprend l'assignation d'une probabilité d'un résultat clinique lié à une lésion rénale subie par le patient présentant un sepsis.

3. Procédé selon la revendication 1, dans lequel l'étape de corrélation comprend l'assignation d'une probabilité que le patient développera la lésion rénale aiguë dans la classe I ou F de RIFLE dans les 48 heures ou 24 heures suivant le moment où l'échantillon est prélevé sur le patient.

4. Procédé selon l'une des revendications 1 ou 2, dans lequel le patient présentant un sepsis est un patient présentant un sepsis sévère, ou

dans lequel le patient présentant un sepsis est un patient en choc septique, ou
dans lequel ladite étape de corrélation comprend le fait d'évaluer si la fonction rénale s'améliore ou s'aggrave ou non chez un patient présentant un sepsis qui a souffert d'une IRA sur la base du résultat d'analyse.

5. Procédé selon l'une des revendications 1 ou 2, dans lequel ladite étape de corrélation comprend l'établissement d'un diagnostic d'occurrence ou de nonoccurrence d'un besoin de transplantation rénale chez ledit patient présentant un sepsis, ou

dans lequel ladite étape de corrélation comprend l'établissement d'un diagnostic d'occurrence ou de nonoccurrence d'un besoin de traitement rénal substitutif chez ledit patient atteint d'un sepsis, ou
dans lequel ladite étape de corrélation comprend la prédiction d'un risque diminué ou accru que le patient présentant un sepsis nécessite un traitement rénal substitutif, ou
dans lequel ladite étape de corrélation comprend la prédiction d'un risque diminué ou accru que le patient présentant un sepsis nécessite une transplantation rénale.

6. Procédé selon l'une des revendications 1 ou 2, dans lequel le patient présentant un sepsis est dans la classe 0 ou R de RIFLE.

7. Procédé selon la revendication 6, dans lequel le patient présentant un sepsis est dans la classe 0 de RIFLE ; ou

dans lequel le patient présentant un sepsis est dans la classe 0 de RIFLE, et ladite étape de corrélation comprend l'assignation d'une probabilité que le patient atteint d'un sepsis atteindra la classe I de RIFLE dans les 72 heures, dans les 48 heures ou dans les 24 heures ; ou
dans lequel le patient présentant un sepsis est dans la classe 0 de RIFLE, et ladite étape de corrélation comprend l'assignation d'une probabilité que le patient atteint d'un sepsis atteindra la classe I ou F de RIFLE dans les 72 heures, dans les 48 heures ou dans les 24 heures ; ou
dans lequel le patient atteint d'un sepsis est dans la classe 0 de RIFLE, et ladite étape de corrélation comprend l'assignation d'une probabilité que le patient présentant un sepsis atteindra la classe F de RIFLE dans les 72 heures, dans les 48 heures ou dans les 24 heures ; ou
dans lequel le patient présentant un sepsis est dans la classe 0 ou R de RIFLE, et ladite étape de corrélation comprend l'assignation d'une probabilité que le patient atteint d'un sepsis atteindra la classe I ou F de RIFLE dans les 72 heures, dans les 48 heures ou dans les 24 heures ; ou
dans lequel le patient présentant un sepsis est dans la classe 0 ou R de RIFLE, et ladite étape de corrélation comprend l'assignation d'une probabilité que le patient atteint d'un sepsis atteindra la classe F de RIFLE dans les 72 heures, dans les 48 heures ou dans les 24 heures.

8. Procédé selon la revendication 6, dans lequel le patient présentant un sepsis est dans la classe R de RIFLE ; ou

dans lequel le patient présentant un sepsis est dans la classe R de RIFLE, et ladite étape de corrélation comprend l'assignation d'une probabilité que le patient atteint d'un sepsis atteindra la classe I de RIFLE dans les 72 heures, dans les 48 heures ou dans les 24 heures ; ou
dans lequel le patient présentant un sepsis est dans la classe R de RIFLE, et ladite étape de corrélation comprend l'assignation d'une probabilité que le patient atteint d'un sepsis atteindra la classe F de RIFLE dans les 72 heures, dans les 48 heures ou dans les 24 heures.

9. Procédé selon l'une des revendications 1 ou 2, dans lequel le patient présentant un sepsis est dans la classe 0, R, ou I de RIFLE, et ladite étape de corrélation comprend l'assignation d'une probabilité que le patient atteint d'un sepsis atteindra la classe F de RIFLE dans les 72 heures, dans les 48 heures ou dans les 24 heures, ou
dans lequel le patient présentant un sepsis est dans la classe I de RIFLE, et ladite étape de corrélation comprend l'assignation d'une probabilité que le patient atteint d'un sepsis atteindra la classe F de RIFLE dans les 72 heures, dans les 48 heures ou dans les 24 heures.

10. Procédé selon l'une des revendications 1 ou 2, dans lequel le patient présentant un sepsis n'a pas connu d'augmentation de 1,5 fois ou plus de la créatinine sérique par rapport à une valeur de référence déterminée avant le moment où l'échantillon de fluide corporel est obtenu, ou

dans lequel le patient présentant un sepsis a une production d'urine d'au moins 0,5 ml/kg/h au cours des 6 heures précédant le moment où l'échantillon de fluide corporel est obtenu, ou
dans lequel le patient présentant un sepsis n'a pas connu d'augmentation de 0,3 mg/dl ou plus de la créatinine sérique par rapport à une valeur de référence déterminée avant le moment où l'échantillon de fluide corporel est obtenu, ou
dans lequel le patient présentant un sepsis (i) n'a pas connu d'augmentation de 1,5 fois ou plus de la créatinine sérique par rapport à une valeur de référence déterminée avant le moment où l'échantillon de fluide corporel est obtenu, (ii) a une production d'urine d'au moins 0,5 ml/kg/h au cours des 6 heures précédant le moment où l'échantillon de fluide corporel est obtenu, et (iii) n'a pas connu d'augmentation de 0,3 mg/dl ou plus de la créatinine sérique par rapport à une valeur de référence déterminée avant le moment où l'échantillon de fluide corporel est obtenu.

11. Procédé selon l'une des revendications 1 ou 2, dans lequel le patient présentant un sepsis n'a pas connu d'augmentation de 2 fois ou plus de la créatinine sérique par rapport à une valeur de référence déterminée avant le moment où l'échantillon de fluide corporel est obtenu, ou

dans lequel le patient présentant un sepsis a une production d'urine d'au moins 0,5 ml/kg/h au cours des 12 heures précédant le moment où l'échantillon de fluide corporel est obtenu, ou
dans lequel le patient présentant un sepsis (i) n'a pas connu d'augmentation de 2 fois ou plus de la créatinine sérique par rapport à une valeur de référence déterminée avant le moment où l'échantillon de fluide corporel est obtenu, et (ii) a une production d'urine d'au moins 0,5 ml/kg/h au cours des 12 heures précédant le moment où l'échantillon de fluide corporel est obtenu.

12. Procédé selon l'une des revendications 1 ou 2, dans lequel le patient présentant un sepsis n'a pas connu d'augmentation de 3 fois ou plus de la créatinine sérique par rapport à une valeur de référence déterminée avant le moment où l'échantillon de fluide corporel est obtenu, ou

dans lequel le patient présentant un sepsis a une production d'urine d'au moins 0,3 ml/kg/h au cours des 24 heures précédant le moment où l'échantillon de fluide corporel est obtenu, et n'a pas souffert d'anurie au cours des 12 heures précédant le moment où l'échantillon de fluide corporel est obtenu, ou

dans lequel le patient présentant un sepsis (i) n'a pas connu d'augmentation de 3 fois ou plus de la créatinine sérique par rapport à une valeur de référence déterminée avant le moment où l'échantillon de fluide corporel est obtenu, et (ii) a une production d'urine d'au moins 0,3 ml/kg/h au cours des 24 heures précédant le moment où l'échantillon de fluide corporel est obtenu, et n'a pas souffert d'anurie au cours des 12 heures précédant le moment où l'échantillon de fluide corporel est obtenu.

13. Procédé selon l'une des revendications 1 ou 2, dans lequel ladite étape de corrélation comprend l'assignation d'une probabilité que dans les 72 heures le patient présentant un sepsis (i) connaîtra une augmentation de 2 fois ou plus de la créatinine sérique et (ii) aura une production d'urine inférieure à 0,5 ml/kg/h sur une période de 12 heures, ou

dans lequel ladite étape de corrélation comprend l'assignation d'une probabilité que dans les 48 heures le patient présentant un sepsis (i) connaîtra une augmentation de 2 fois ou plus de la créatinine sérique et (ii) aura une production d'urine inférieure à 0,5 ml/kg/h sur une période de 12 heures, ou

dans lequel ladite étape de corrélation comprend l'assignation d'une probabilité que dans les 24 heures le patient présentant un sepsis (i) connaîtra une augmentation de 2 fois ou plus de la créatinine sérique et (ii) aura une production d'urine inférieure à 0,5 ml/kg/h sur une période de 12 heures, ou

dans lequel ladite étape de corrélation comprend l'assignation d'une probabilité que dans les 72 heures le patient présentant un sepsis connaîtra une augmentation de 2 fois ou plus de la créatinine sérique, ou

dans lequel ladite étape de corrélation comprend l'assignation d'une probabilité que dans les 72 heures le patient présentant un sepsis aura une production d'urine inférieure à 0,5 ml/kg/h sur une période de 12 heures, ou

dans lequel ladite étape de corrélation comprend l'assignation d'une probabilité que dans les 48 heures le patient présentant un sepsis connaîtra une augmentation de 2 fois ou plus de la créatinine sérique, ou

dans lequel ladite étape de corrélation comprend l'assignation d'une probabilité que dans les 48 heures le patient présentant un sepsis aura une production d'urine inférieure à 0,5 ml/kg/h sur une période de 12 heures, ou

dans lequel ladite étape de corrélation comprend l'assignation d'une probabilité que dans les 24 heures le patient présentant un sepsis connaîtra une augmentation de 2 fois ou plus de la créatinine sérique, ou

dans lequel ladite étape de corrélation comprend l'assignation d'une probabilité que dans les 24 heures le patient présentant un sepsis aura une production d'urine inférieure à 0,5 ml/kg/h sur une période de 12 heures, ou

dans lequel ladite étape de corrélation comprend l'assignation d'une probabilité que dans les 72 heures le patient atteint d'un sepsis (i) connaîtra une augmentation de 3 fois ou plus de la créatinine sérique et (ii) aura une production d'urine inférieure à 0,3 ml/kg/h sur une période de 24 heures ou une anurie sur une période de 12 heures.

14. Procédé selon l'une des revendications 1 ou 2, dans lequel ladite étape de corrélation comprend l'assignation d'une probabilité que dans les 48 heures le patient présentant un sepsis (i) connaîtra une augmentation de 3 fois ou plus de la créatinine sérique, et (ii) aura une production d'urine inférieure à 0,3 ml/kg/h sur une période de 24 heures ou une anurie sur une période de 12 heures, ou

dans lequel ladite étape de corrélation comprend l'assignation d'une probabilité que dans les 24 heures le patient atteint d'un sepsis (i) connaîtra une augmentation de 3 fois ou plus de la créatinine sérique et (ii) aura une production d'urine inférieure à 0,3 ml/kg/h sur une période de 24 heures ou une anurie sur une période de 12 heures, ou

dans lequel ladite étape de corrélation comprend l'assignation d'une probabilité que dans les 72 heures le patient présentant un sepsis connaîtra une augmentation de 3 fois ou plus de la créatinine sérique, ou

dans lequel ladite étape de corrélation comprend l'assignation d'une probabilité que dans les 72 heures le patient présentant un sepsis aura une production d'urine inférieure à 0,3 ml/kg/h sur une période de 24 heures ou une anurie sur une période de 12 heures, ou

dans lequel ladite étape de corrélation comprend l'assignation d'une probabilité que dans les 48 heures le patient présentant un sepsis connaîtra une augmentation de 3 fois ou plus de la créatinine sérique, ou

dans lequel ladite étape de corrélation comprend l'assignation d'une probabilité que dans les 48 heures le patient présentant un sepsis aura une production d'urine inférieure à 0,3 ml/kg/h sur une période de 24 heures ou une anurie sur une période de 12 heures, ou

dans lequel ladite étape de corrélation comprend l'assignation d'une probabilité que dans les 24 heures le patient présentant un sepsis connaîtra une augmentation de 3 fois ou plus de la créatinine sérique, ou

dans lequel ladite étape de corrélation comprend l'assignation d'une probabilité que dans les 24 heures le patient présentant un sepsis aura une production d'urine inférieure à 0,3 ml/kg/h sur une période de 24 heures ou une anurie sur une période de 12 heures.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61682213
- WO 2010036342 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- **YAGMUR et al.** *Clin. Biochem.,* 2012, vol. 45 (1-2), 82-87
- **BONE et al.** *Chest,* 1992, vol. 101, 1644-53
- **LLEWELYN ; COHEN.** *Int. Care Med.,* 2001, vol. 27, S10-S32
- **JAIMES et al.** *Int. Care Med,* 26 June 2003, vol. 29, 1368-71
- **ZARJOU ; AGARWAL.** *J. Am. Soc. Nephrol.,* 2011, vol. 22, 999-1006
- **RONCO et al.** *Clin. J. Am. Soc. Nephrol.,* 2008, vol. 3, 531-44
- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** Curr Opin Nephrol Hypertens. 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **HANLEY, J. A. ; MCNEIL, B.J.** The meaning and use of the area under a receiver operating characteristic (ROC) curve. *Radiology,* 1982, vol. 143, 29-36